# EUROPEAN PATENT APPLICATION

(11) **EP 3 845 249 A1**
(43) Date of publication of application: **07.07.2021**
(21) Application number: 19806493.3
(22) Date of filing: 22.05.2019
(51) Int. Cl.: A61K 47/26, A61K 47/34, A61K 9/127

(54) **TARGETED NANOPREPARATION OF MANNOSE, AND PREPARATION THEREFOR AND APPLICATION THEREOF**

(30) Priority: 25.05.2018 CN 201810511368
(71) Applicant: Chengdu Ribocure Pharmatech Company Limited, Wuhou District Chengdu Sichuan 610000 (CN)
(72) Inventor: SONG, Xiangrong, Chengdu, Sichuan 610000 (CN); WEI, Yuquan, Chengdu, Sichuan 610000 (CN)
(74) Representative: Gill, Siân Victoria
(86) International application number: PCT/CN2019/088001
(87) International publication number: WO 2019/223728

(57) **Abstract**

The present invention relates to the field of pharmaceutical preparations, in particular, to a mannose modified targeting nano-preparations, a composition for preparing nano-preparations, a targeting element, a targeting vector, a prepared targeting drug and a preparation method and the application thereof. The described nano-preparations with targeting function is composed of the targeting ligand mannose and its derivatives, nano-preparations and main drug components, and the described targeting material is linked with the spacer material, and then linked with the nano-preparations material to prepare the nano-preparations. The targeting nano-preparations in the present invention has good targetability of mannose receptor, can effectively bond with mannose receptor on target cell. Moreove, the preparation method has universality, can be used for synthesizing a variety of targeting nano-preparations, and is conducive to purification and characterization.

## Description

### Priority Application

This application claims the priority of the invention patent application 201810511368.2 "A mannose modified targeting nano-preparations" in China, which was submitted on May 25, 2018, and the patent application for priority invention is incorporated in full text by reference.

### Technical field

The present invention relates to the field of medicine, in particular, to a mannose modified targeting nano-preparations, a composition for preparing nano-preparations, a targeting element, a targeting vector, a prepared targeting drug and a preparation method and application thereof.

### Background technology

In recent years, targeted drug delivery system mediated by drug receptors has attracted wide attention, and it can improve its specific targetability and solve the problems of wide drug distribution and serious side effects in vivo. Lectin receptors are a type of glycoproteins, glycolipids or glycoconjugates distributed on the surface of cell membrane, which can specifically recognize and bind some glycosyls. Mannose is the most important and efficient endocytic lectin receptor. Its functions include eliminating endogenous molecules, promoting antigen presentation, regulating cell activation and transportation. It is also closely related to tumor immune escape and metastasis. It is mainly expressed in macrophages, dendritic cells and tumor cells, and can specifically recognize the glycosyl molecules of mannose. As the most potential target group, mannose has many advantages such as non-toxicity, non-immunogenicity, good biocompatibility and biodegradability, and it can be widely used for glycosylation modification of drug delivery system.

Liposomes and nanoparticles are the most classic nano-vectors for targeted drug delivery, and their targeting effect is based on the different uptake of particle size in various organs, and selective aggregation in lung and other lymphoid organs, so as to improve the therapeutic effect of anti-tumor drugs in various organs. Its preparation is simple and has the characteristics such as controlled release, non-immunogenicity and improving efficacy, etc. However, after liposomes and nanoparticles achieve targetability based on macrophage endocytosis, their stability and local uptake efficiency of lesion in the targeted delivery process are relatively low, and their targetability is required to be further improved. Therefore, the research highlights in the field of anti-tumor focus on improving the targetability of nanoparticles and developing novel targeting vector with the continuous development of biopreparations, material science and nanotechnology. At present, there are many different types of nano-targeted drug delivery.

For instance, when liposomes are used as drug targeting vectors, they can be divided into physical targeting liposomes and molecular targeting liposomes according to the principle of their targeting effect, which has been fully explored currently. Among them, physical targeting liposomes include pH sensitive liposome (PLP), photosensitive liposome, magnetic liposome, thermosensitive liposome and so on. Taking photosensitive liposomes as an example, Yang et al. constructed pcCPP-NGR-LP by linking photosensitive cell osmotic peptide pcCPP and a short peptide containing aspartate-glycine-arginine residue (NGR) on the surface of the liposome. This photosensitive liposome can promote cell uptake, effectively silence c-mycc gene and delay the growth of human fibrosarcoma. Although the above different forms of liposomes enhance the targeting effect to a certain extent, there are some shortcomings. For PH sensitive liposomes, the cumulative toxicity of liposomes in the liver and spleen has not been solved. The organism release feasibility and long-term storage stability of photosensitive and magnetic liposomes still need urgent solutions. Thermosensitive liposomes can kill tumor cells directly, but heating for a long time can also damage normal tissues. In addition, molecular targeting liposomes also have some problems such as poor drug targeting delivery and poor absorption of target organs in vivo.

Summing up the above, the primary problem to be solved in the research and development of targeting drugs is the stability of nano-vectors in the delivery process. Common nanoparticles or liposomes are easy to be removed by the endothelial system due to their lack of active targetability (some may have passive targetability), which reduces the utilization rate and may cause unnecessary toxic and side effects. Furthermore, the key of the research is that the drugs directly enter the cells and quickly have medical effect via effective ligands after the drugs reach the target cells.

### Detailed description of the invention

One of the objectives of the present invention is to provide a nano-preparation, and improve the targeability of the nano-preparations by modifying with mannose and its derivatives.

The second objective of the present invention is to provide the application of the above targeting nano-preparations.

The present invention provides a targeting nano-preparation modified by mannose and its derivatives, which is composed of targeting ligand mannose and its derivatives, nano-preparations and main drug components, and can be used for vaccine delivery, targeted cancer therapy and treatment of arteriosclerosis and various inflammatory diseases.

Specifically, the mannose derivative of the targeting nano-preparations provided by the present invention is one or more of mannoside, mannosamine, mannan, etc.

Specifically, the mannose derivative of the targeting nano-preparations provided by the present invention are one or more of methyl-D-mannoside, 1-α formylmethyl-mannopyranoside, 4-aminophenyl-α-D-mannopyranoside, 4-nitrophenyl-α-D-mannopyranoside, 4-methylumbelliferyl-α-D-mannopyranoside, mannose-6-phosphate and carbamoyl-D-mannose, mannosamine, mannan,etc.

Specifically, the targeting nano-preparations provided by the present invention is one or more of liposomes, emulsions, nanogels, core-shell nanoparticles, HDL nanoparticles, solid lipid nanoparticles, polymeric micelles, etc.

Specifically, the main drug component of the targeting nano-preparations provided by the present invention comprises one or more of small molecule drugs, protein polypeptide drugs or gene drug.

Specifically, small molecule drugs of the main drug component of the targeting nano-preparations provided by the present invention is small effective molecule which can be encapsulated in the nano-preparations and used for targeted cancer therapy, treatment of arteriosclerosis and various inflammatory diseases. For example, antitumor drug taxanes (Taxol, docetaxel, harringtonine, 7-epitaxol), camptothecins (camptothecin, SN38, irinotecan, 9-aminocamptothecin, 9-nitrocamptothecin, etc.), vinblastines (vinblastine, vincristine, vindesine, vinorelbine, vinflunine, etc.), doxorubicin, epirubicin, daunorubicin, epirubicin, amphotericin, methotrexate, cytarabine, 5-fluorouracil, mitoxantrone or its derivatives, gefitinib, noscapine, cisplatin, carboplatin, oxaliplatin, carmustine, quercetin, etc. The main component protein polypeptide drugs are interleukin (for example, IL-1, IL-la, IL-2, IL-3, IL-4, IL-5, IL-6, etc.), various growth factors (for example, fibroblast growth factor, liver growth factor, vascular endothelial growth factor, hematopoietic growth factor, etc.), interferon (for example, IFN α, IFN β, IFN γ), tumor necrosis factor (for example, TNF α, TNF β), integrin, monoclonal antibody, enzyme, insulin, etc. The main component gene drug is DNA, plasmid, mRNA, siRNA, shRNA, microRNA, etc. When the targeting nano-preparations is used for immunotherapy, the main drug component can also encapsulate the immune adjuvant in addition to the drug used for immunotherapy to enhance the immune effect.

Specifically, the content of the targeting ligand mannose and its derivatives in the targeting nano-preparations provided by the present invention is 0.05% - 40%.

Preferably, the content of the targeting ligand mannose and its derivatives in the targeting nano-preparations provided by the present invention is 1%-10%.

More preferably, the content of the targeting ligand mannose in the targeting nano-preparations provided by the present invention is 2% - 5%.

Specifically, for the targeting nano-preparations provided by the present invention, the modification method of the targeting ligand mannose and its derivatives is as follows: after preparing the nano-preparations, the mannose and its derivatives are adsorbed or coupled to the surface of the nano-preparations; alternatively, the mannose and its derivatives are coupled with the vector material for preparing the nano-preparations, and then the nano-preparations is prepared.

Specifically, in the present invention, the mannose and its derivatives are adsorbed on the surface of the nano-preparations by solvent evaporation, high pressure homogenization or microemulsion method.

Specifically, for the targeting nano-preparations provided by the present invention, the mannose and its derivatives modifying nano-preparations are composed of a single mannose and its derivatives and/or 2-10 mannose and its derivatives.

Specifically, for the targeting nano-preparations provided by the present invention, mannose and its derivatives are adsorbed on the surface of the nano-preparations by solvent evaporation, high pressure homogenization or microemulsion method.

Specifically, for the targeting nano-preparations provided by the present invention, the coupling mode of mannose and its derivatives with the nano-preparations or the vector material for preparing the nano-preparations is direct coupling or coupling through a spacer.

Specifically, for the targeting nano-preparations provided by the present invention, the mannose and its derivatives are coupled with the nano-preparations or the vector material for preparing the nano-preparations through a spacer, and the spacer is one or more of glutaraldehyde, ethylenediamine, malonic acid, short alkyl chain (for example, - CH2-CH2 -), PEG, amino acid, dipeptide, oligopeptide, polypeptide, stearoyl, palmitoyl, etc. Specifically, the spacer PEG has hydroxyl groups at both ends or amino groups at one end at least, with a molecular weight of 100-5000. Preferably, the molecular weight of the spacer PEG is 100-2000. The spacer amino acids are arginine, asparagine, aspartic acid, glutamic acid, glutamine, lysine, serine, threonine and tyrosine. The amino acids at both ends of dipeptide, oligopeptide and polypeptide are selected from the following: arginine, asparagine, aspartic acid, glutamic acid, glutamine, lysine, serine, threonine and tyrosine; the intermediate amino acids are selected from 20 kinds of arbitrary amino acids (for example, RGD).

In particular, for the targeting nano-preparations provided by the present invention, the vector materials for preparing nano-preparations have active amino or hydroxyl groups, which are pharmaceutically acceptable for preparing micelles, emulsions, nanogels, core-shell nanoparticles, HDL nanoparticles, solid lipid nanoparticles, liposomes, polymeric micelles, etc. Specifically, the vector material is one or more of lipid, polylactic acid-glycolic acid copolymer (PLGA), polylactic acid (PLA), polycaprolactone (PCL), polylysine (PLL), polyethyleneimine (PEI), hyaluronic acid (HA), chitosan, gelatin, poloxamer, stearyl alcohol, etc. Specifically, the lipid materials for preparing liposomes and core-shell nanoparticles are one or more of cholesterol, egg yolk lecithin, soybean lecithin, cephalin, sphingomyelin, PC (phosphatidylcholine), EPG (egg phosphatidylglycerol), SPG (soy phosphatidylglycerol), distearoyl-Phosphatidylethanolamine (DSPE), dipalmitoyl-phosphatidylethanolamine (DPPE), dipalmitoyl-phosphatidylcholine (DPPC), dioleoyl-phosphatidylcholine (DOPC), distearoyl-phosphatidylcholine (DSPC), dimyristoyl-phosphatidylcholine (DMPC), dilinoleyl-phosphatidylcholine (DLPC), dioleoyl-phosphatidylglycerol (DOPG), dipalmitoyl-phosphatidylglycerol (DPPG), dimyristoyl-phosphatidylcholine (DMPC), dilauroyl-phosphatidylglycerol (DLPG), cetyltrimethylammonium bromide (CTAB), dimethyl bis octadecyl ammonium bromide (DDAB), 1,2-dioleoyl-3-trimethylammonium propane (chloride salt) (DOTAP), etc. The solid lipid material for preparing solid lipid nanoparticles is one or more of stearic acid, cholesterol, glyceryl monostearate, glyceryl distearate, glyceryl tristearate, glyceryl behenate, glyceryl laurate, glyceryl palmitate stearate, behenic acid monoglyceride, behenic acid diglyceride, behenic acid triglyceride, glyceryl trimyristate, glyceryl citrate, stearyl alcohol, palmitic acid, myristic acid, behenic acid and lauric acid.

The third objective of the present invention is to provide a material composition for preparing a drug vector with specific targeting, and the composition can be used for preparing a drug vector with strong targeting.

To achieve the above objective, the technical scheme of the present invention is as follows:
The composition for preparing nano-preparations with targeting function comprises targeting material and basic nano-preparation material, and the targeting material is mannose and / or mannose derivatives.

Further, the mannose derivative is mannoside and / or mannosamine and / or mannan.

Furthermore, the mannose derivatives are methyl-D-mannoside, 1-α formylmethyl-mannopyranoside, 4-aminophenyl-α-D-mannopyranoside, 4-nitrophenyl-α-D-mannopyranoside, 4-methylumbelliferyl-α-D-mannopyranoside, mannose-6-phosphate and carbamoyl-D-mannose.

Further, the weight percentage of the targeting material in the composition is 0.05-40%.

Preferably, the weight percentage of the targeting material in the composition is 1-10%.

Further, the composition is composed of the targeting material, the basic nano-preparation material and the spacer material which makes the distance between the targeting material and the basic nano-preparation material. The spacer material is one or more of glutaraldehyde, ethylenediamine, malonic acid, short alkyl chain, PEG, amino acid, dipeptide, oligopeptide, polypeptide, stearoyl and palmitoyl.

Specifically, the spacer is one or more of glutaraldehyde, ethylenediamine, malonic acid, short alkyl chain (for example, - CH2-CH2 -), PEG, amino acid, dipeptide, oligopeptide, polypeptide, etc. Specifically, the spacer amino acids are arginine, asparagine, aspartic acid, glutamine, lysine, serine, threonine and tyrosine. The amino acids at both ends of dipeptide, oligopeptide and polypeptide are selected from the following: arginine, asparagine, aspartic acid, glutamic acid, glutamine, lysine, serine, threonine and tyrosine; the intermediate amino acids are selected from 20 kinds of arbitrary amino acids (for example, RGD).

Furthermore, the basic nano-preparation material is a composition for preparing liposomes, emulsions, nanogels, core-shell nanoparticles, HDL nanoparticles, solid lipid nanoparticles and polymeric micelles.

Specifically, the basic nano-preparation material is a composition for preparing the nano-preparation material, which may be the nano-preparations material mentioned in "Modern Pharmaceutical Preparation Technology".

Specifically, the basic nano-preparation material is one or more of lipid, polylactic acid-glycolic acid copolymer (PLGA), polylactic acid (PLA), polycaprolactone (PCL), polylysine (PLL), polyethyleneimine (PEI), hyaluronic acid (HA), chitosan, gelatin, poloxamer, stearyl alcohol, etc.

Specifically, the lipid materials for preparing liposomes and core-shell nanoparticles are one or more of cholesterol, egg yolk lecithin, soybean lecithin, cephalin, sphingomyelin, PC (phosphatidylcholine), EPG (egg phosphatidylglycerol), SPG (soy phosphatidylglycerol), distearoyl-phosphatidylethanolamine (DSPE), dipalmitoyl-phosphatidylethanolamine (DPPE), dipalmitoyl-phosphatidylcholine (DPPC), dioleoyl-phosphatidylcholine (DOPC), distearoyl-phosphatidylcholine (DSPC), dimyristoyl-phosphatidylcholine (DMPC), dilinoleyl-phosphatidylcholine (DLPC), dioleoyl-phosphatidylglycerol (DOPG), dipalmitoyl-phosphatidylglycerol (DPPG), dimyristoyl-phosphatidylcholine (DMPC), dilauroyl-phosphatidylglycerol (DLPG), cetyltrimethylammonium bromide (CTAB), dimethyl bis octadecyl ammonium bromide (DDAB), 1,2-dioleoyl-3-trimethylammonium propane (chloride salt) (DOTAP), etc.

Specifically, the solid lipid material for preparing solid lipid nanoparticles is one or more of stearic acid, cholesterol, glyceryl monostearate, glyceryl distearate, glyceryl tristearate, glyceryl behenate, glyceryl laurate, glyceryl palmitate stearate, behenic acid monoglyceride, behenic acid diglyceride, behenic acid triglyceride, glyceryl trimyristate, glyceryl citrate, stearyl alcohol, palmitic acid, myristic acid, behenic acid and lauric acid.

Further, the spacer material is PEGn, where n = 100-5000.

Preferably, the n is 200, 400, 1000 or 2000.

Furthermore, it is composed of the following components based on part by weight: mannose: 1; PEG: 0.5-56; cholesterol: 2-11.

Preferably, it is composed of the following components based on part by weight: mannose: 1; PEG: 10-25; cholesterol: 4-8.

Furthermore, it is composed of the following components by the mole fraction: mannose: 1; PEG: 1-5; cholesterol: 1-5.

Preferably, it is composed of the following components by the mole fraction: mannose: 1; PEG: 1-3; cholesterol: 1-3.

The fourth objective of the present invention is to provide a targeting vector and a targeting drug. The targeting vector can deliver a variety of medicinal effective ingredients to the target cells, and has strong targetabilty.

The present invention adopts the following scheme to achieve the above objective:
The targeting vector for drug delivery is prepared by using the described composition.

Further, the drug is a small molecule drug and / or a protein polypeptide drug and / or a gene drug.

The small molecule drug is a small effective molecule which can be encapsulated in nano-preparations for targeted cancer therapy, treatment of arteriosclerosis and various inflammatory diseases.

In some Embodiments, the mRNA encoding the specific tumor antigen can be encapsulated in the targeting nano-preparations vector, and then introduced into the somatic cell, and the antigen protein is synthesized through the expression system of the host cell to induce the immune response of the host immune system to the tumor antigen, so as to realize the function of tumor prevention and treatment. Moreover, the targeting nano-preparations carrying the mRNA have better tumor targetability and are beneficial to the treatment of tumor. In additon, anti-tumor chemical and biological drugs, drugs for the treatment of arteriosclerosis and inflammatory diseases can also be encapsulated in the vector of targeting nano-preparations to achieve more accurate targeted therapy.

Specifically, the small molecule drug includes but is not limited to PTX, DOX and quercetin, and the protein polypeptide drug includes but is not limited to albumin, and the gene drug includes but is not limited to mRNA and siRNA.

More specifically, the small molecule drug is small effective molecule which can be encapsulated in the nano-preparations and used for targeted cancer therapy, treatment of arteriosclerosis and various inflammatory diseases. For example, antitumor drug taxanes (Taxol, docetaxel, harringtonine, 7-epitaxol), camptothecins (camptothecin, SN38, irinotecan, 9-aminocamptothecin, 9-nitrocamptothecin, etc.), vinblastines (vinblastine, vincristine, vindesine, vinorelbine, vinflunine, etc.), doxorubicin, epirubicin, daunorubicin, epirubicin, amphotericin, methotrexate, cytarabine, 5-fluorouracil, mitoxantrone or its derivatives, gefitinib, noscapine, cisplatin, carboplatin, oxaliplatin, carmustine, quercetin, etc. The main component protein polypeptide drugs are interleukin (for example, IL-1, IL-la, IL-2, IL-3, IL-4, IL-5, IL-6, etc.), various growth factors (for example, fibroblast growth factor, liver growth factor, vascular endothelial growth factor, hematopoietic growth factor, etc.), interferon (for example, IFN α, IFN β, IFN γ), tumor necrosis factor (for example, TNF α, TNF β), integrin, monoclonal antibody, enzyme, insulin, etc. The main component gene drug is DNA, plasmid, mRNA, siRNA, shRNA, microRNA, etc. When the targeting nano-preparations is used for immunotherapy, the main drug component can also encapsulate the immune adjuvant in addition to the drug used for immunotherapy to enhance the immune effect.

The drug is encapsulated by the targeting vector.

Further, the drug is a small molecule drug and / or a protein polypeptide drug and / or a gene drug.

Specifically, the small molecule drug includes but is not limited to PTX, DOX and quercetin, the protein polypeptide drug includes but is not limited to albumin, and the gene drug includes but is not limited to mRNA and siRNA.

Further, the drug can be any pharmaceutically acceptable dosage form.

Specifically, the dosage form includes one or more pharmaceutically acceptable vectors, diluents or excipients, which are added in the appropriate steps in the preparation process. The term "Pharmaceutically Acceptable" used in the present invention indicates that within reasonable medical judgment, the compounds, raw materials, compositions and / or dosage forms are suitable for contact with patients' tissues without excessive toxicity, irritation, allergy or other problems and complications symmetrical with a reasonable benefit / risk ratio, and are effectively applied to the intended use.

The preparations can be used for any suitable administration route, such as oral (including mouth cavity or sublingual), rectal, nasal, local (including mouth cavity, sublingual or transcutaneous), vaginal or extra-gastrointestinal (including subcutaneous, intradermal, intramuscular, intra-articular, intrasynovial, intrasternal, intrathecal, intralesional, intravenous or subepidermal injection or infusion) administration. Such preparations can be prepared by any known method in the field of pharmaceutical science, for example, mix the active ingredient with a vector or excipient. Oral, local or injection administration is preferred. The preparations suitable for oral administration is provided by independent unit, such as solution or suspensions in aqueous or non-aqueous liquid; capsules or tablets; powders or granules; edible foam preparations or foaming preparations , etc.

The fifth objective of the present invention is to provide a preparation method of the targeting vector, which can be used for industrial preparation of the targeting vector.

The present invention adopts the following scheme to achieve the above objective:
The method for preparing the targeting vector is as followed: nano drug-delivery vector is prepared by using the basic nano-preparation material, and then attach the targeting material to the surface of the nano drug-delivery vector.

The method of attaching the targeting material to the surface of the nano drug-delivery vector includes but is not limited to the preparation of nano-drug vector by solvent volatilization, film dispersion, ultrasonic dispersion, injection, reverse evaporation, high pressure homogenization and microemulsion method, and then the solution of the targeting material is mixed with it to make the targeting material attached to its surface. For the above method for preparing nano-drug vector, please refer to "Modern Pharmaceutical Preparation Technology"

Specifically, the solvent evaporatio is a method for preparing microsphere. The simple process is as followed: first prepare a polymer solution with appropriate concentration, then emulsify the polymer solution in the continuous phase, gradually volatilize the solvent to obtain microsphere preparations. Subsequently, add targeting material to the microsphere preparations to make it adsorbd on the surface of the microsphere preparations.

The high-pressure homogenization method is that the material is ejected at an extremely high flow rate through the homogenization valve under high pressure to impact the ring, and the material is ultra-fine comminuted, dispersed and emulsified to form nano-particles through the cavitation, impact and shear effect. In this process, the surface area of the material is increased, and the targeting material can be attached to the surface after addition of the targeting material.

The film evaporation method is as followed: dissolve the phospholipid material in the organic solvent, and evaporate the organic solvent under the reduced pressure state. After the film is formed on the inner wall of the bottle, add water or PBS solution to repeatedly stir. After homogenizing and ultrasonic treatment of the washed film, the nano-preparations can be obtained, and the targeting material can attach to the surface of it when addition of the targeting material,

The sixth objective of the present invention is to provide a method for improving the targetabilty of the targeting vector, which can significantly enhance the targetabilty of the targeting vector.

The present invention adopts the following scheme to achieve the above objective:
The method for improving the targetabilty of the targeting vector includes the following steps:
A Synthesize targeting element
Synthesize targeting element by using the targeting material and the spacer material;
B Prepare the targeting vector
Prepare the nano-preparations by using the basic nano-preparation material, and then link the targeting element in step A with the nano drug-delivery vector to obtain the targeting vector, alternatively, first synthesize the spacer material with the basic nano-preparation material, and then link with the targeting material to obtain the targeting vector.

Further, the distance between the targeting element and the nano drug-delivery vector is 0.2-100nm.

Preferably, the distance between the targeting element and the nano drug-delivery vector is 0.3-10nm.

The seventh objective of the present invention is to provide a method for delivering drugs, which can efficiently deliver drugs to the destination.

The present invention adopts the following scheme to achieve the above objective:
The method for delivering the effective ingredient to the target cell is as followed: place the effective ingredient in the targeting vector and deliver it.

Further, the target cells contain mannose receptors.

Furthermore, the target cells include but are not limited to macrophages, dendritic cells and tumor cells.

The eighth objective of the present invention is to provide a composition with a targeting element, which can be used to prepare a drug delivery vector with strong targetability.

The present invention adopts the following technical scheme to achieve the above objective:
The composition with a targeting element is composed of targeting material and spacer material, and the targeting material is mannose and / or mannose derivative.

Further, the mannose derivative is mannoside and / or mannosamine and / or mannan.

Furthermore, the mannose derivative are methyl-D-mannoside, 1-α formylmethyl-mannopyranoside, 4-aminophenyl-α-D-mannopyranoside, 4-nitrophenyl-α-D-mannopyranoside, 4-methylumbelliferyl-α-D-mannopyranoside, mannose-6-phosphate and carbamoyl-D-mannose.

Further, the spacer material is one or more of glutaraldehyde, ethylenediamine, malonic acid, short alkyl chain, PEG, amino acid, dipeptide, oligopeptide, polypeptide, stearoyl and palmitoyl.

Specifically, the spacer group is one or more of glutaraldehyde, ethylenediamine, malonic acid, short alkyl chain (for example, - CH2-CH2 -), PEG, amino acid, dipeptide, oligopeptide, polypeptide, etc. Specifically, the spacer amino acids are arginine, asparagine, aspartic acid, glutamic acid, glutamine, lysine, serine, threonine and tyrosine. The amino acids at both ends of dipeptide, oligopeptide and polypeptide are selected from the following: arginine, asparagine, aspartic acid, glutamic acid, glutamine, lysine, serine, threonine and tyrosine; the intermediate amino acids are selected from 20 kinds of arbitrary amino acids (for example, RGD).

Furthermore, the molar ratio of the spacer material to the targeting material is 10-1:1.

Preferably, the molar ratio of the spacer material to the targeting material is 5:1.

Furthermore, the molar ratio of mannose to PEG 100 is 1:1.

The ninth objective of the present invention is to provide a targeting element, which can be used to prepare drug delivery vector with strong targetability

The present invention adopts the following scheme to achieve the above objective:
The targeting element is prepared by using the composition.

Furthermore, the targeting element is shown in formula I, wherein R1 is - C₅H₈O₂, -C₂H₇N₂, -C₃H₃O₄, -O(CH₂CH₂O)nH, -C₂H₃O₂NR' or -C₂H₃ONR', -C₄H₅O₃N₂R₁' R₂' or -C₄H₅O₂N₂R₁' R₂', -C₃n+1H₃n+2On+2Nn+1R₁' R₂' or -C₃n+1H₃n+2On+1Nn+1R₁' R₂', -C₁₈H₃₅O,C₁₆H₃₁O, -H , -CHO,-C₆H₄NH₂, - C₆H₄NO₂, -CONH₂, -CH₂C₆CCHCO₂CH₃, both R₁' and R₂' are R' groups of any natural amino acid side chain; R₂ is - CH₃ or - PO₃H₂; R₃ is - H; R₄ is - H; R₅ is - H or - NHCOCH₃,

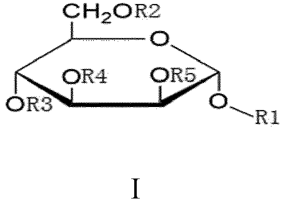

Furthermore, R1 is polyethylene glycol, and its structure is as follows: 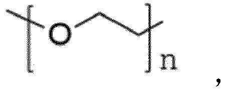 where, n = 1 ∼ 5000, the corresponding molecular weight is 40 * n.

The tenth objective of the present invention is to provide a targeting vector, which can deliver drugs to target cells.

The present invention adopts the following scheme to achieve the above objective:
The targeting vector contains the targeting element.

Further, the targeting vector is composed of a targeting element and nano-preparations. The nano-preparations are liposomes, emulsions, nanogels, core-shell nanoparticles, HDL nanoparticles, solid lipid nanoparticles and polymeric micelles.

Specifically, the nano-preparations material may be mentioned in "Modern Pharmaceutical Preparation Technology".

Specifically, the material of the nano-preparations is one or more of lipid, polylactic acid-glycolic acid copolymer (PLGA), polylactic acid (PLA), polycaprolactone (PCL), polylysine (PLL), polyethyleneimine (PEI), hyaluronic acid (HA), chitosan, gelatin, poloxamer, stearyl alcohol, etc.

Specifically, the lipid materials for preparing liposomes and core-shell nanoparticles are are one or more of cholesterol, egg yolk lecithin, soybean lecithin, cephalin, sphingomyelin, PC (phosphatidylcholine), EPG (egg phosphatidylglycerol), SPG (soy phosphatidylglycerol), distearoyl-Phosphatidylethanolamine (DSPE), dipalmitoyl-phosphatidylethanolamine (DPPE), dipalmitoyl-phosphatidylcholine (DPPC), dioleoyl-phosphatidylcholine (DOPC), distearoyl-phosphatidylcholine (DSPC), dimyristoyl-phosphatidylcholine (DMPC), dilinoleyl-phosphatidylcholine (DLPC), dioleoyl-phosphatidylglycerol (DOPG), dipalmitoyl-phosphatidylglycerol (DPPG), dimyristoyl-phosphatidylcholine (DMPC), dilauroyl-phosphatidylglycerol (DLPG), cetyltrimethylammonium bromide (CTAB), dimethyl bis octadecyl ammonium bromide (DDAB), 1,2-dioleoyl-3-trimethylammonium propane (chloride salt) (DOTAP), etc.

Specifically, the solid lipid material for preparing solid lipid nanoparticles is one or more of stearic acid, cholesterol, glyceryl monostearate, glyceryl distearate, glyceryl tristearate, glyceryl behenate, glyceryl laurate, glyceryl palmitate stearate, behenic acid monoglyceride, behenic acid diglyceride, behenic acid triglyceride, glyceryl trimyristate, glyceryl citrate, stearyl alcohol, palmitic acid, myristic acid, behenic acid and lauric acid.

Further, the single targeting vector is linked with 1-100000 targeting elements.

Preferably, the single targeting vector is linked with 1-1000 targeting elements.

Further, the distance between the head of the targeting element and the nano-preparations is 0.2-100nm.

Furthermore, the distance between the head of the targeting element and the nano-preparations is 0.3-10nm.

Further, the target head is mannoside and / or mannosamine and / or mannan.

The tenth objective of the present invention is to provide a method for linking the targeting element to the nano-preparations. The method has universality, which can be used for synthesizing a variety of targeting nano-preparations, and is conducive to purification and characterization.

The present invention adopts the following scheme to achieve the above objective:
The method for linking the targeting element to the nano-preparations is characterized in that the targeting material and the spacer material are linked, and then linked with the nano-preparations material to obtain the nano-preparations.

Further, it includes the following steps:
1) Diethylene glycol, p-toluenesulfonyl chloride and triethylamine are dissolved in DCM and reacts at room temperature, and Compound 1 is obtained by column chromatography;
2) Compound 1 is dissolved in DCM with pentaacetylated mannose and its derivatives and boron trifluoride diethyl etherate. The Compound 2 is obtained by column chromatography;
3) Compound 2 and sodium azide are dissolved in DMF and Solid compound 3 is obtained by silica gel column chromatography;
4) Compound 3 is dissolved in methanol solution of sodium methoxide, reacts at room temperature and Compound 4 is obtained by concentration;
5) Cholesterol, bromopropyne and sodium-hydrogen are dissolved in the mixed solution of ether and DMF, reacts at room temperature. Compound 5 is obtaimned by silica gel column chromatography;
6) Compound 4, Compound 5 and cuprous iodide are dissolved in DMF and reacts for 24 h at room temperature. The Solid compound 6 of targeting vector is obtained by silica gel column chromatography.

In some embodiments, diethylene glycol, p-toluenesulfonyl chloride and triethylamine were dissolved in DCM and reacted for 24 h at room temperature and the product ToSOC₂H₄OC₂H₄OH (Compound 1) was obtained by column chromatography. Compound 1 was dissolved in DCM with pentaacetylated mannose and its derivatives and boron trifluoride diethyl etherate (BF₃·Et₂O), reacted for 24 h at room temperature and Aco-M-OC₂H₄OC₂H₄OTos (Compound 2) was obtained by column chromatography, where M was mannose and its derivatives. Compound 2 and sodium azide were dissolved in DMF , reacted at 60°C for 24 h, and the solid product ACO-M-OC₂H₄OC₂H₄N₃ (Compound 3) was obtained by silica gel column chromatography. Compound 3 was dissolved in methanol solution of sodium methoxide, reacted for 3 h at room temperature, and the product M-OC₂H₄OC₂H₄N₃ (Compound 4) was obtain by concentration. Cholesterol, bromopropyne and sodium-hydrogen were dissolved in the mixture of ether and DMF, reacted for 24 h at room temperature, and the solid product Chol-CH₂CCH (Compound 5) was obtained by silica gel column chromatography. Compound 4, Compound 5 and cuprous iodide were dissolved in DMF, reacted for 24 h at room temperature, and the solid product M-PEGₙ-Chol (Compound 6) was obtained by silica gel column chromatography.

The 12th objective of the present invention is to provide a method for improving the drug delivery capacity of the targeting vector as descripbed in the claim.

The present invention adopts the following scheme to achieve the above objective:
The method for improving the drug delivery capacity of the targeting vector as claimed is to encapsulate the drug in the targeting vector for delivery.

Further, the drug is a small molecule drug and / or a protein polypeptide drug and / or a gene drug

Specifically, the small molecule drug is effective small molecule which can be encapsulated in the nano-preparations and used for the targeted cancer therapy, treatment of arteriosclerosis and various inflammatory diseases.

In some Embodiments, the mRNA encoding the specific tumor antigen can be encapsulated in the targeting nano-preparation vector, and then introduced into the somatic cell, and the antigen protein was synthesized through the expression system of the host cell to induce the immune response of the host immune system to the tumor antigen, so as to realize the function of tumor prevention and treatment. Moreover, the mRNA-loaded targeting nano-preparations have better tumor targetability and are beneficial to the treatment of tumor. In additon, anti-tumor chemical and biological drugs, drugs for the treatment of arteriosclerosis and inflammatory diseases can also be encapsulated in the vector of targeting nano-preparations to achieve more accurate targeted therapy.

Specifically, the small molecule drug includes but is not limited to PTX, DOX and quercetin; the protein polypeptide drug includes but is not limited to albumin, and the gene drug includes but is not limited to mRNA and siRNA.

More specifically, the small molecule drug is effective small molecule which can be encapsulated in the nano-preparations for targeted cancer therapy, treatment of arteriosclerosis and the various inflammatory diseases. For example, antitumor drug taxanes (Taxol, docetaxel, harringtonine, 7-epitaxol), camptothecins (camptothecin, SN38, irinotecan, 9-aminocamptothecin, 9-nitrocamptothecin, etc.), vinblastines (vinblastine, vincristine, vindesine, vinorelbine, vinflunine, etc.), doxorubicin, epirubicin, daunorubicin, epirubicin, amphotericin, methotrexate, cytarabine, 5-fluorouracil, mitoxantrone or its derivatives, gefitinib, noscapine, cisplatin, carboplatin, oxaliplatin, carmustine, quercetin, etc. The main component protein polypeptide drugs are interleukin (for example, IL-1, IL-la, IL-2, IL-3, IL-4, IL-5, IL-6, etc.), various growth factors (for example, fibroblast growth factor, liver growth factor, vascular endothelial growth factor, hematopoietic growth factor, etc.), interferon (for example, IFN α, IFN β, IFN γ), tumor necrosis factor (for example, TNF α, TNF β), integrin, monoclonal antibody, enzyme, insulin, etc. The main component gene drug is DNA, plasmid, mRNA, siRNA, shRNA, microRNA, etc. When the targeting nano-preparations is used for immunotherapy, the main drug component can also encapsulate the immune adjuvant in addition to the drug used for immunotherapy to enhance the immune effect.

Further, the drug can be any pharmaceutically acceptable dosage form.

Specifically, the dosage form includes one or more pharmaceutically acceptable vectors, diluents or excipients, which are added in the appropriate steps in the preparation process. The term "Pharmaceutically Acceptable" in the present invention indicates that within reasonable medical judgment, the compounds, raw materials, compositions and / or dosage forms are suitable for contact with patients' tissues without excessive toxicity, irritation, allergy or other problems and complications symmetrical with a reasonable benefit / risk ratio, and are effectively applied to the intended use.

The preparations can be used for any suitable administration route, such as oral (including mouth cavity or sublingual), rectal, nasal, local (including mouth cavity, sublingual or transcutaneous), vaginal or extra-gastrointestinal (including subcutaneous, intradermal, intramuscular, intra-articular, intrasynovial, intrasternal, intrathecal, intralesional, intravenous or subepidermal injection or infusion) administration. Such preparations can be prepared by any known method in the field of pharmaceutical science, for example, mix the active ingredient with a vector or excipient. Oral, local or injection administration is preferred. The preparations suitable for oral administration is provided by independent unit, such as solution or suspensions in aqueous or non-aqueous liquid; capsules or tablets; powders or granules; edible foam preparations or foaming preparations , etc.

'Further, the targeting vector can deliver the drug to a target cell containing mannose receptor.

The 13th objective of the present invention is to provide a compound containing the targeting vector.

The present invention adopts the following scheme to achieve the above objective:
The compound is formed by the targeting vector and the receptor.

Further, the targeting vector delivers the drug.

Further, the receptor is a mannose.

The 14th objective of the present invention is to provide a method for improving the efficiency of the transfection reagent, which can significantly enhance the efficiency of the transfection reagent.

The present invention adopts the following scheme to achieve the above objective:
The method for improving the efficiency of the transfection reagent is as followed: link the targeting element with the vector for preparing the transfection reagent to prepare the transfection reagent with high efficiency.

Transfection refers to the process in which eukaryotic cells acquire new genetic markers due to the incorporation of exogenous DNA. The development of DNA transfection technology has greatly affected the modern molecular biology. Gene transfection is not only an important tool to study transgene and gene expression, but also a key step in gene therapy. The ideal gene transfection reagent should have the following characteristics: efficient transfection; safety; low cytotoxicity; simple method; time-saving and economical.

The 15th objective of the present invention is to provide the application of the targeting element.

The invention adopts the following scheme to achieve the above objective:
The targeting element is used as a target oil phase.

The targeting element is used as a target aqueous phase.

Specifically, it is based on "like dissolves like". Those that are not easily soluble in water belong to the oil phase, conversely, to the aqueous phase. More simply, the substance is mixed with water, and it belongs to the aqueous phase when forming a transparent solution, and to the oil phase when stratified or turbid.

### The beneficial effects of the present invention are as follows:

1) The targeting nano-preparations, the composition of the nano-preparations, the targeting element, the targeting vector and the targeted drug provided by the present invention have good targetability of mannose receptor, and can effectively bind with the mannose receptor on the target cell;
2) The preparation of the targeting element and targeting vector provided by the present invention is easy, has low cost and universality, and can synthesize a number of targeting nano-preparations, and is conducive to purification and characterization;
3) The spacer material provided by the present invention can produce a certain distance between the targeting material and the basic nano-preparation material, so that the targeting material can be exposed to the surface of the targeting nano-preparations to have high targetability of mannose receptor;
4) The targeting nano-preparations show good profile, similar to spherical shape with small size, have good serum stability and low cytotoxicity, and can be used in the targeted drug delivery system (chemical and biological drugs. Moreover, the transfection efficiency is significantly higher than that of commercialized Lipo 3K, and it can be used as a transfection reagent for scientific research and commercial application.

### Description of figures

Figure 1: the NMR verification results of mannose -PEG₁₀₀ Chol
Figure 2: the particle size of the liposome of Formulation 1
Figure 3: the electric potential of the liposome of Formulation 1
Figure 4: the TEM image of the liposome of Formulation 1
Figure 5: the results of liposome transfection
Figure 6: the expression of GFP in DC cells under fluorescence microscope
Figure7: the histogram of transfection efficiency analysis
Figure 8: the stability of mannose-PEG₁₀₀₀-Chol
Figure 9: the transfection efficiency of mannose-PEG₁₀₀₀-Chol at 4 °C
Figure 10: the stability of mannose-PEG₁₀₀₀-Chol in serum
Figure 11: the cytotoxicity of mannose-PEG₁₀₀₀-Chol and Lipo 3K to DC detected by flow cytometry
Figure 12: the statistical analysis histogram of the cytotoxicity of mannose-PEG₁₀₀₀-Chol and Lipo 3K to DC detected by flow cytometry
Figure 13: the study of DC2.4 uptake targeting nanoparticles
Figure 14: the uptake of mRNA targeted liposome complexes in BMDCs under fluorescence microscope
Figure 15: the data analysis of mRNA targeted liposome complex uptake in BMDCs under fluorescence microscope
Figure 16: the in vivo antitumor effect of the vaccine prepared with mRNA-loaded liposome containing mannose, polyethylene glycol-400 and cholesterol
Figure 17: the in vivo antitumor effect of the vaccine prepared with mRNA-loaded liposome containing mannose, polyethylene glycol-400 and cholesterol: the body weight monitoring of mice
Figure 18: the in vivo antitumor effect of the vaccine prepared with mRNA-loaded liposome containing mannose, polyethylene glycol-400 and cholesterol: the tumor volume of mice on Day 28
Figure 19: the in vivo antitumor effect of m/MP400-LPX in each group
Figure 20: the survival time of mice immunized with m/MP400-LPX
Figure 21: the uptake of mRNA-loaded targeted liposome complexes with different ligand chains in DC2.4
Figure 22: data analysis of the uptake of mRNA-loaded targeted liposome complexes with different ligand chain lengths in DC2.4
Figure 23: the transfection effect of DC2.4 on targeted liposome complexes with different ligand chain lengths
Figure 24: data analysis of the transfection effect of DC2.4 on targeted liposome complexes with different ligand chain lengths
Figure 25: the transfection effect of BMDCs on targeted liposome complexes with different ligand chain lengths
Figure 26: data analysis of the transfection effect of BMDCs on targeted liposome complexes with different ligand chain lengths
Figure 27: the in vivo antitumor effect of targeted liposome complexes with different ligand chain lengths
Figure 28: the in vivo anti-tumor study of targeted liposome complexes with different ligand chain lengths - the body weight monitoring of mice
Figure 29: the in vivo anti-tumor study of targeted liposome complexes with different ligand chain lengths - the tumor volume of mice on Day 28
Figure 30: the data analysis of in vivo anti-tumor effect of targeted liposome complexes with different ligand chain lengths
Figure 31: the survival time of mice immunized with targeted liposome complexes with different ligand chain lengths

### Specific implementation mode

The listed embodiments are intended to better explain the present invention, but the content of the invention is not limited to the Embodiments. Therefore, the non-essential improvement and adjustment to the implementation scheme performed by the technical personnel familiar with the field according to the above invention content are still within the protection scope of the present invention.

### Embodiment 1

In view of the shortcomings of the existing nano-preparations, a targeting nano-preparations modified by mannose and its derivatives was designed.

In the present implementation mode, the nano-preparation is composed of a targeting ligand mannose and its derivatives, a nano-preparations and main drug components.

In the present implementation mode, the targeting ligand is mannose.

In other implementation modes, the targeting ligand may be mannose derivatives, one or more of the above mentioned mannoside, mannosamine, mannan, etc. Specifically, mannose derivatives are one or more of methyl-D-mannoside, 1-α formylmethyl-mannopyranoside, 4-aminophenyl - α-D-mannopyranoside, 4-nitrophenyl-α-D-mannopyranoside, 4-methylumbelliferyl-α-D-mannopyranoside, mannose-6-phosphate and carbamoyl-D-mannose, mannosamine and mannan, etc.

In the present implementation mode, the nano-preparation is liposome, HDL nanoparticles, solid lipid nanoparticles and polymeric micelles.

In other implementation modes, the nano-preparations can be one or more of emulsions, nanogels, core-shell nanoparticles, etc.

In the present implementation mode, the main drug component encapsulated in the nano-preparations is one or more of small molecule drugs, protein polypeptide drugs or gene drug.

In the present implementation mode, the nano-preparations is modified by a single mannose and its derivatives and / or 2-10 mannose and its derivatives.

In the present implementation mode, the targeting ligand mannose and its derivatives are coupled with the vector material for preparing the nano-preparations, and then the nano-preparations are prepared.

In other implementation modes, the targeting ligand mannose and its derivatives can be made into nano-preparations, and then mannose and its derivatives are adsorbed or coupled to the surface of the nano-preparations.

In the present implementation mode, mannose and its derivatives are coupled with the nano-preparations or the vector material for preparing the nano-preparations through the spacer PEG.

In other implementation modes, mannose and its derivatives are directly coupled with the nano-preparations or the vector material for preparing the nano-preparations or coupled through other spacers, which can be one or more of the above-mentioned glutaraldehyde, ethylenediamine, malonic acid, short alkyl chain (for example -CH2-Ch2-), PEG, amino acid, dipeptide, oligopeptide, polypeptide, etc. Specifically, the spacer amino acids are arginine, asparagine, aspartic acid, glutamic acid, glutamine, lysine, serine, threonine and tyrosine. The amino acids at both ends of dipeptide, oligoderm and polypeptide are selected from the following: arginine, asparagine, aspartic acid, glutamic acid, glutamine, lysine, serine, threonine and tyrosine; the intermediate amino acids are selected from 20 arbitrary amino acids (for example, RGD).

In the present implementation mode, the unmodified ordinary PEG with both hydroxyl ends can be used, or one end of PEG is aminated at least, and one end of PEG is linked with DSPE, cholesterol and palmitic acid.

In other implementation modes, one end of PEG can also be linked with other pharmaceutically acceptable vector materials with active amino or hydroxyl groups for preparing liposomes, HDL nanoparticles, solid lipid nanoparticles, polymeric micelles, etc. Specifically, the vector materials are one or more of lipid, polylactic acid-glycolic acid copolymer (PLGA), polycaprolactone (PCL), polylysine (PLL), polyethyleneimine (PEI), hyaluronic acid (HA), chitosan, gelatin, poloxamer, stearic alcohol egg yolk lecithin, soybean lecithin, cephalin, sphingomyelin, PC (phosphatidylcholine), EPG (egg phosphatidylglycerol), SPG (soy phosphatidylglycerol), dipalmitoyl-phosphatidylethanolamine (DPPE), dipalmitoyl-phosphatidylcholine (DPPC), dioleoyl-phosphatidylcholine (DOPC), distearoyl-phosphatidylcholine (DSPC), dimyristoyl-phosphatidylcholine (DMPC), dilinoleyl-phosphatidylcholine (DLPC), dioleoyl-phosphatidylglycerol (DOPG), dipalmitoyl-phosphatidylglycerol (DPPG), dimyristoyl-phosphatidylcholine (DMPC), dilauroyl-phosphatidylglycerol (DLPG), cetyltrimethylammonium bromide (CTAB), dimethyl bis octadecyl ammonium bromide (DDAB), 1,2-dioleoyl-3-trimethylammonium propane (chloride salt) (DOTAP), stearic acid, cholesterol, glyceryl monostearate, glyceryl distearate, glyceryl tristearate, glyceryl behenate, glyceryl laurate, glyceryl palmitate stearate, behenic acid monoglyceride, behenic acid diglyceride, behenic acid triglyceride, glyceryl trimyristate, glyceryl citrate, stearyl alcohol, myristic acid, behenic acid and lauric acid, etc.

In the present implementation mode, the prepared nano-preparations is delivered into the body by intravenous injection and subcutaneous injection.

In other implementation modes, the prepared nano-preparations can also be delivered into the body by intraperitoneal injection and intramuscular injection.

The mRNA involved in the embodiment is obtained under conventional conditions, such as the conditions described in Molecular Cloning: A Laboratory Manual (Third Edition, J. Sambrook et al.), or the conditions recommended by the manufacturer or purchased.

The following are specific embodiments.

### Embodiment 1: Synthesis of vector material

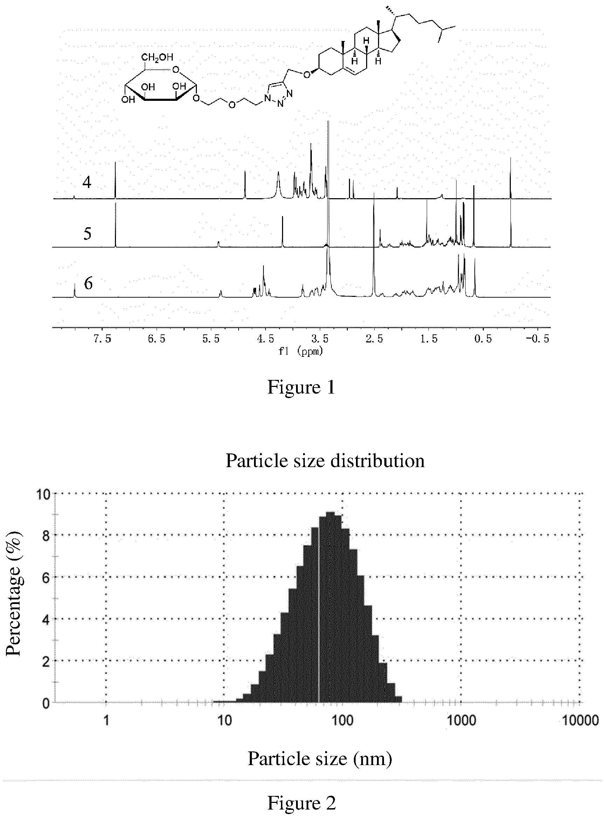

Mannose-PEGn-Chol: Taking the synthesis of mannose-PEG100-Chol as an example, other PEGs with different lengths are also synthesized according to this synthetic route. Diethylene glycol, p-toluenesulfonyl chloride and triethylamine were dissolved in DCM, reacted at room temperature for 24 h, and the product ToSOC₂H₄OC₂H₄OH (Compound 1) was obtained by silica gel column chromatography. Compound 1 was dissolved in DCM with pentaacetylated mannose and boron trifluoride diethyl etherate (BF₃·Et₂O), reacted for 24 h at room temperature and Aco-Mannose-OC2H4OC2H4OTos (Compound 2) was obtained by silica gel column chromatography. Compound 2 and sodium azide were dissolved in DMF , reacted at 60°C for 24 h, and the solid product Aco-Mannose-OC2H4OC2H4N3 (Compound 3) was obtained by silica gel column chromatography. Compound 3 was dissolved in methanol solution of sodium methoxide, reacted for 3 h at room temperature, and the product Mannose-OC2H4OC2H4N3 (Compound 4) was obtain by concentration. Cholesterol, bromopropyne and sodium-hydrogen were dissolved in the mixture of ether and DMF, reacted for 24 h at room temperature, and the solid product Chol-CH2CCH (Compound 5) was obtained by silica gel column chromatography. Compound 4, Compound 5 and cuprous iodide were dissolved in DMF, reacted for 24 h at room temperature, and the solid product Mannose-Chol (Compound 6) was obtained by silica gel column chromatography. The synthetic route was shown in reaction formula I (the synthetic route of mannose-PEG1000-Chol was shown in reaction formula II). The chemical shift δ (ppm) of each characteristic hydrogen proton in Figure 1 was analyzed. 5.30-5.42 ppm (1) was the hydrogen shift of =CH- in cholesterol, and 7.92ppm (1) corresponded to hydrogen in N-CH=C. The presence of these characteristic peaks indicated that Compound 4 was linked to the fragment of Compound 5. The 1H-NMR results showed that mannose-PEG100-Chol was successfully coupled.

Mannose-PEG2000-DSPE: DSPE-PEG200-NH2 can be purchased directly. 5mg of mannose was dissolved in 5ml of ethanol. 5mg DSPE-PEG-NH2 was dissolved in 5ml chloroform-ethanol mixed solvent (9:1, V/V), and 0.5ml mannose ethanol solution was added dropwise under nitrogen protection; after mixed homogenously, 100µl triethylamine was added, with stirring at 40°C for 12h. After the reaction, the organic solvent was removed by rotary evaporation under reduced pressure, deionized water was added to redissolve. The unreacted mannose was removed by dialyzing, and the product mannose-PEG2000-DSPE was obtained by cryodesiccation.

Synthesis of mannose-PEG2000-palmitic acid: Dissolve FmocNH-PEG-COOH in water, add EDC•HCl and sulfo-NHS, and stir at room temperature for 10 minutes. Add the dissolved 2-aminoethyl-α-D-mannopyranoside in water to the above reaction system, and react at room temperature for 48 hours, and the product (Compound 1) was obtained by silica gel column chromatography. Dissolve Compound 1 in DCM, add 1-octanediol, and react for several hours, and the product (compound 2) was obtained by silica gel column chromatography. Compound 2 and palmitic acid were added to a mixed solvent of ether and DMF, and the reaction was carried out at room temperature for several hours. The solid product mannose-PEG2000-palmitic acid was obtained by silica gel column chromatography, and it structural formula was as shown below:

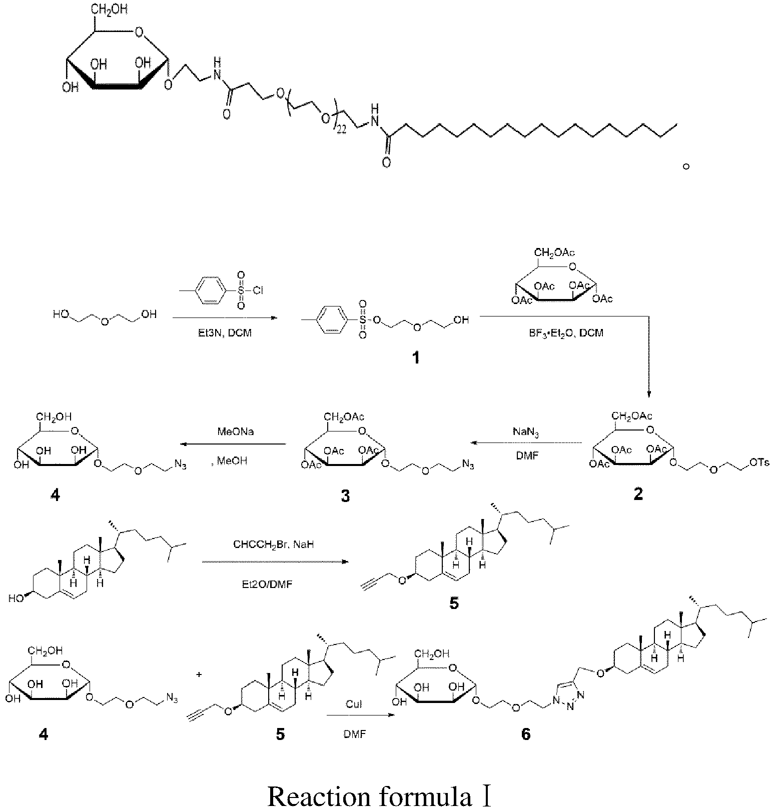

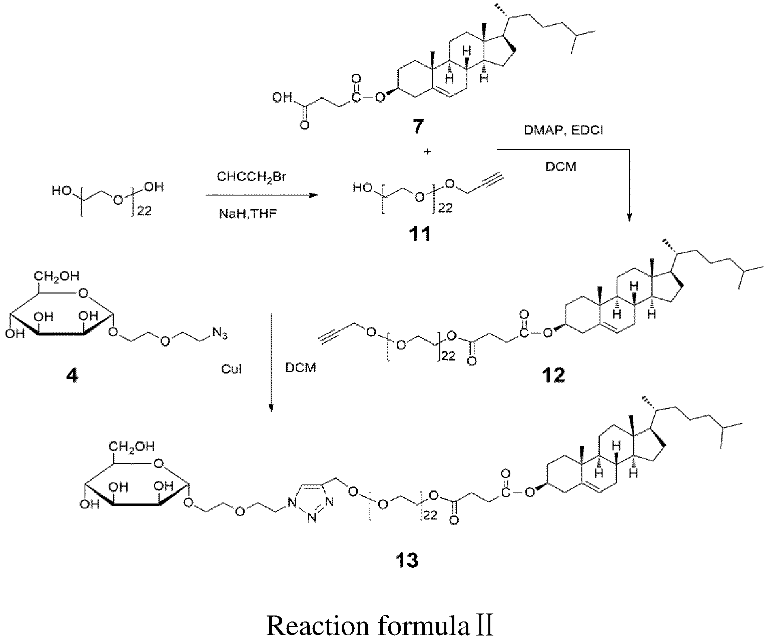

### Embodiment 2: Preparation of liposomes

### Formulation composition

| Formulation | DOTAP | DOPC | Chol | Mannose -PEG100-Chol | Mannose -PEG 1000-Chol | Mannose -PEG2000-Chol | Mannose -PEG5000-Chol |
|---|---|---|---|---|---|---|---|
| Formulation 1 | 50 | 10 | 40 | 0 | 0 | 0 | 0 |
| Formulation 2 | 50 | 10 | 35 | 5 | 0 | 0 | 0 |
| Formulation 3 | 50 | 10 | 35 | 0 | 5 | 0 | 0 |
| Formulation 4 | 50 | 10 | 35 | 0 | 0 | 5 | 0 |
| Formulation 5 | 50 | 10 | 35 | 0 | 0 | 0 | 5 |

| Formulation | DOTAP | DSPE-PE G2000 | Chol | Mannose -PEG2000-DSPE | | Main drug | |
|---|---|---|---|---|---|---|---|
| Formulation 6 | 50 | 10 | 35 | 5 | | pGFP | |
| Formulation 7 | 50 | 10 | 35 | 2 | | PTX | |
| Formulation 8 | 50 | 10 | 35 | 1 | | Albumin | |
| Formulation 9 | 50 | 10 | 35 | 0 | | PTX | |
| Formulation 10 | 50 | 10 | 0 | 0 | | pGFP | |

| | | | | | | | |
|---|---|---|---|---|---|---|---|
| Note: the main drug of Formulation 1 ∼ 5 is GFP- mRNA. | | | | | | | |

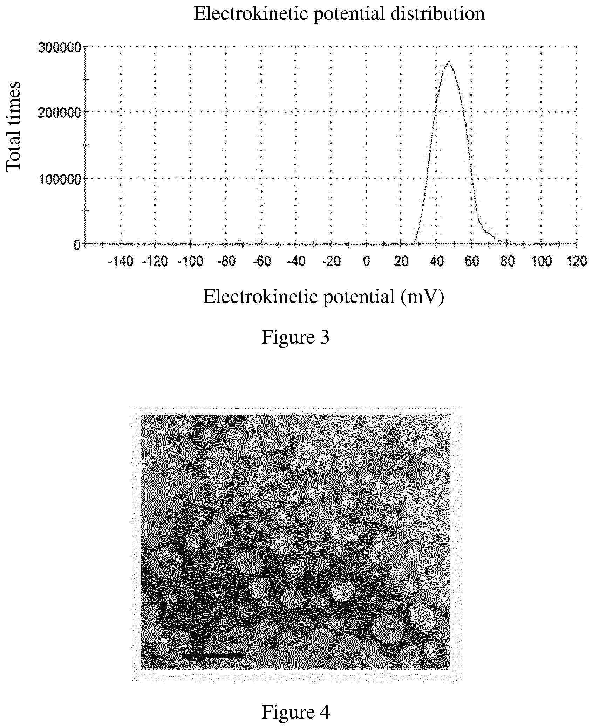

**Preparation method 1:** the different proportions of phospholipids (Main drug PTX was added together with phospholipids) were added into a round-bottom flask according to the above formulation, and dissolved by chloroform / ethanol = 1:1 (V / V). After the organic solvent was removed by rotary vaporation under reduced pressure, a thin film was formed on the inner wall of the bottle, and PBS 7.4 buffer solution was added for hydration, followed by treatment with 100 W ultrasonic for 3 min to obtain liposomes. The blank liposome was incubated with pGFP at room temperature to encapsulate pGFP. The obtained liposomes were characterized, and results were shown in Figure 2-4.

**Preparation method 2** (Formulation 8): the different proportions of phospholipids were dissolved with chloroform/ether according to the above formulation, and then the aqueous solution of albumin was added to obtain the emulsion by ultrasonic treatment. The organic solvent was removed by rotary evaporation. Subsequently, PBS 7.4 buffer solution was added to rehydrate the liposome.

### Embodiment 3: Preparation of solid lipid nanoparticles

Solid lipid nanoparticles are nano-structured vectors based on lipid as the framework material, which have the characteristics such as physiological compatibility, cell affinity and targeting, etc. In the present embodiment, addition of mannose modified lipid improved the targetability of solid lipid nanoparticles.

### Formulation composition

| | Oil phase (10ml) | | | | Aqueous phase (35ml) | | Main drug (DOX) |
|---|---|---|---|---|---|---|---|
| Formulation | Mannose-PE G2000-palmitic acid | Glyceryl tristearate | Glyceryl monostearate | Soybeans lecithin | Poloxamer 188 | Tween 80 | |
| Formulation 11 | 0 | 0 | 40mg | 60mg | 0 | 2% | 10mg |
| Formulation 12 | 0 | 0 | 40mg | 60mg | 3% | 0 | 10mg |
| Formulation 13 | 20mg | 0 | 80mg | 0mg | 3% | 0 | 10mg |
| Formulation 14 | 20mg | 80mg | 0 | 0mg | 2% | 0 | 10mg |
| Formulation 15 | 50mg | 50mg | 0 | 0mg | 2% | 0 | 10mg |
| Formulation 16 | 50mg | 50mg | 0 | 0mg | 4% | 0 | 10mg |

**Preparation method:** the accurately weighed drugs and lipid materials according to the formulation were dissolved in ethanol to form the oil phase by stirring at a constant temperature in water bath; the formulated amount of surfactant was dissolved in purified water to form the aqueous phase by heating at a constant temperature in water bath; under stirring conditions, the oil phase was injected into the aqueous phase to emulsify and concentrate at a constant temperature. After the emulsion was concentrated to a certain volume, pour it into cold water at a constant temperature of 4°C for solidifying with stirring, followed by filtering with 0.45 µm membrane.

### Embodiment 4: Preparation of HDL nanoparticles

HDL is a kind of lipoprotein with heterogeneous composition, density and particle size. ApoA-I is the most abundant apolipoprotein in HDL, and its hydrophobic core transports cholesterol in the body. Recombinant high density lipoprotein is formed by the recombination of ApoA-I endogenously isolated or synthesized in vitro, phospholipid and cholesterol, and it is similar to endogenous HDL in biochemical characteristics and functions. High density lipoprotein (HDL) has many excellent characteristics such as high safety, high efficiency of delivery (delivery drug in three ways: core-embedding, lipophilic drug embedding and recombinant high density lipoprotein core; surface insertion, and lipophilic drug insertion into the phospholipid monolayer; protein bonding), targetability. In the present embodiment, mannose ligand was added to the prepared HDL nanoparticles, and the targetability of the nanoparticles was further improved.

### Formulation composition

| Formulation | DOTAP | DOPC | DSPE-PEG2000 | Mannose-PEG2000- DSPE | Chol | Mannose -PEG1000-Chol | ApoA-I | Main drug |
|---|---|---|---|---|---|---|---|---|
| Formulation 17 | 60mg | 15mg | 45mg | 0mg | 0mg | 0mg | 1mg | DOXlOmg |
| Formulation 18 | 0mg | 120mg | 0mg | 0mg | 0mg | 0mg | 1mg | DOXlOmg |
| Formulation 19 | 0mg | 100mg | 0mg | 20mg | 0mg | 0mg | 1mg | DOXlOmg |
| Formulation 20 | 0mg | 100mg | 0mg | 20mg | 0mg | 0mg | 1.5mg | DOXlOmg |
| Formulation 21 | 50mg | 10mg | 0mg | 0mg | 20mg | 20mg | 1mg | DOXlOmg |

| Formulation | DOTAP | DSPE-P EG2000 | PLGA | Chol | Mannose-PEG1000-Chol | | ApoA-I | Main drug |
|---|---|---|---|---|---|---|---|---|
| Formulation 22 | 50 | 10 | 10 | 30 | 10 | | 0.5mg | siRNA/PT X |
| Formulation 23 | 50 | 10 | 10 | 30 | 10 | | 1mg | siRNA/PT X |
| Formulation 24 | 50 | 10 | 10 | 30 | 10 | | 1.5mg | siRNA/PT X |
| Formulation 25 | 50 | 10 | 10 | 30 | 10 | | 2mg | siRNA/PT X |

**Preparation method:** it is similar to the preparation of liposomes, and the method suitable for preparing liposomes can be used for the preparation of HDL nanoparticles.

**Preparation method 1:** the different proportions of phospholipid (main drug DOX liposoluble is added simultaneously) were added into a round-bottom flask according to the above formulation, and dissolved by chloroform. After the organic solvent was removed by rotary vaporation under reduced pressure, a layer of film was formed on the inner wall of the bottle. PBS7.4 buffer solution was added for hydration, followed by 100W ultrasonic treatment for 3min to obtain liposome. The formulated amount of ApoA-I was added into liposome injectable suspension slowly for stationary incubation at 4 °C for 24h, followed by dialyzing.

**Preparation method 2:** the different proportions of phospholipid were added into a round-bottom flask according to the above formulation, and dissolve by chloroform. After the organic solvent was removed by rotary vaporation under reduced pressure, a layer of film was formed on the inner wall of the bottle. PLGA/PTX nano-particle injectable suspension was added to obtain liposome by 100W ultrasonic treatment for 3min. The formulated amount of ApoA-I was added into liposome injectable suspension slowly for stationary incubation at 4 °C for 24h, followed by dialyzing.

### Embodiment 5: Preparation of polymeric micelles

### Formulation composition

| Formulation | Mannose-PEG-DS PE | DSPE | DOPC | DSPC | DMPC | Main drug |
|---|---|---|---|---|---|---|
| Formulation 26 | 20 | 60 | 20 | 0 | 0 | Quercetin |
| Formulation 27 | 20 | 60 | 0 | 20 | 0 | DOX |
| Formulation 28 | 20 | 60 | 35 | 0 | 20 | PTX |
| Formulation 29 | 20 | 60 | 10 | 35 | 0 | DOX |
| Formulation 30 | 50 | 0 | 50 | 0 | 0 | DOX |

**Preparation method:** the vector material and the main drug were dissolved in chloroform (other solvents such as ethanol or the mixed solvent of ethanol and chloroform can be selected according to the dissolution). The organic solvent was removed by rotary vaporation under reduced pressure, and a thin film was formed on the inner wall of the bottle. PBS 7.4 buffer solution was added for hydration.

### Embodiment 6: Uptake of targeting nanoparticles

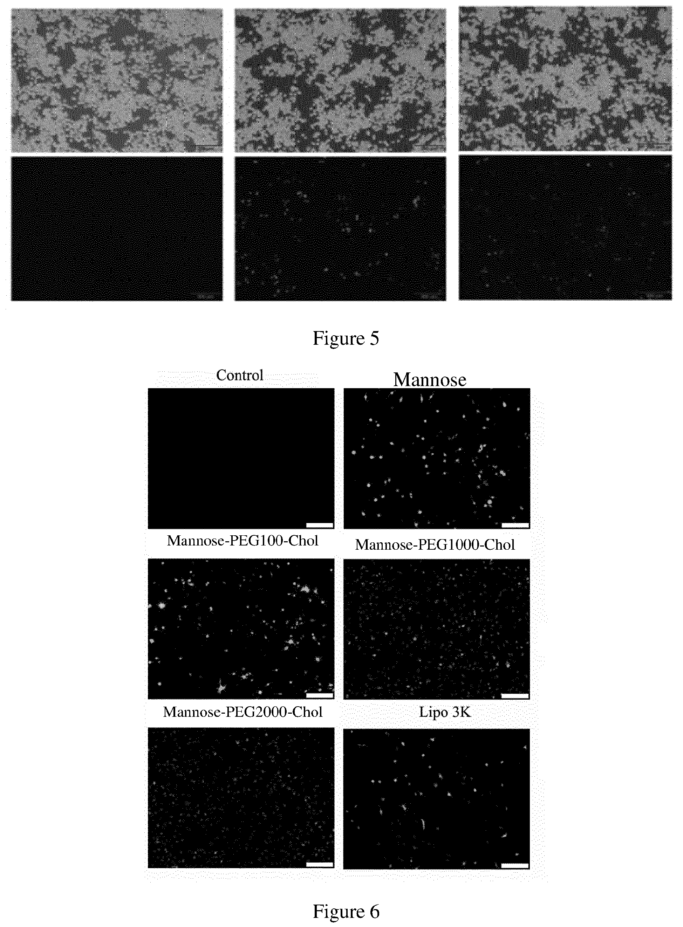

12 hours before transfection, Raw264.7 cells in the plate were blown away, and the medium was re-suspended and counted. 1ml cell suspension with a density of 10 × 10⁴ / ml was placed into each well of 24-well plate, and incubated in the incubator for about 12 hours, to be standby. After discarding the medium, lipopolysaccharide (LPS) with final concentration of 100 ng / mL was added into each well, and incubated at 37 °C for 12 hours to stimulate macrophages. After discarding the medium, different preparations with final concentration of 25 ng / mL (Formulation 1 and Formulation 3) were separately added into each well, and incubated in dark at 37 °C for 2 hours to investigate the uptake of different preparations by macrophages. After reaching incubation time, discard the medium and wash twice with cold PBS; the cells in 24-well plate were collected by PBS, and then washed with PBS once (1200 rpm, 5 min), subsequently analyzed by flow cytometry. The results were shown in Figure 5 (from left to right: control, Formulation 3 and Formulation 1). It was found that the addition of targeting ligand mannose-PEG1000-Chol can significantly increase the transfection efficiency of the cells.

### Embodiment7: Materials

| No. | Targeting material (mol) | Spacer material (mol) | Basic nano-preparation materials (mol) |
|---|---|---|---|
| 1 | Mannose 0.01mol | PEG₁₀₀0.1mol | Lipid 0.1mol |
| 2 | Mannose 0.01mol | PEG₂₀₀0.05mol | Lipid 0.5mol |
| 3 | Mannose 0.01mol | PEG₄₀₀0.02mol | Lipid 0.3mol |
| 4 | Mannose 0.01mol | PEG₁₀₀₀0.01mol | Lipid 0.06mol |
| 5 | Mannose 0.01mol | PEG₂₀₀₀0.1mol | Lipid 0.1mol |
| 6 | Methyl-D-mannoside 0.01mol | Glutaraldehyde 0.01mol | PLGA0.1mol |
| 7 | 1 - α-formylmethyl-mannopyranoside 0.01mol | Ethylenediamine 0.06mol | PLA0.2mol |
| 8 | 4-nitrophenyl - α-D-mannopyranoside 0.01mol | Malonic acid 0.08mol | PCL0.5mol |
| 9 | 4-methylumbelliferyl - α-D-mannopyranoside 0.01mol | Amino acid 0.04mol | PLL0.1mol |
| 10 | Mannose-6-phosphate, carbamoyl-D-mannose 0.01mol | Peptide 0.02mol | PEI0.05mol |
| 11 | D-mannosamine hydrochloride 0.01mol | PEG₁₀₀0.01mol | HA0.1mol |
| 12 | N-acetyl-D-mannosamine 0.01mol | PEG₁₀₀0.01mol | Chitosan 0.3mol |
| 13 | Mannan 0.01mol | PEG₁₀₀₀0.05mol | Gelatin 0.5mol |
| 14 | Mannose 0.01mol | PEG₁₀₀₀0.08mol | Poloxamer 0.6mol |
| 15 | Mannose 0.01mol | PEG₁₀₀₀0.01mol | Stearyl alcohol 0.1mol |

In the present embodiment, the lipid material is one or more of cholesterol, egg yolk lecithin, soybean lecithin, cephalin, sphingomyelin, PC (phosphatidylcholine), EPG (egg phosphatidylglycerol), SPG (soy phosphatidylglycerol), distearoyl-Phosphatidylethanolamine (DSPE), dipalmitoyl-phosphatidylethanolamine (DPPE), dipalmitoyl-phosphatidylcholine (DPPC), dioleoyl-phosphatidylcholine (DOPC), distearoyl-phosphatidylcholine (DSPC), dimyristoyl-phosphatidylcholine (DMPC), dilinoleyl-phosphatidylcholine (DLPC), dioleoyl-phosphatidylglycerol (DOPG), dipalmitoyl-phosphatidylglycerol (DPPG), dimyristoyl-phosphatidylcholine (DMPC), dilauroyl-phosphatidylglycerol (DLPG), cetyltrimethylammonium bromide (CTAB), dimethyl bis octadecyl ammonium bromide (DDAB), 1,2-dioleoyl-3-trimethylammonium propane (chloride salt) (DOTAP), etc.

In the present embodiment, the spacer amino acid is one or more of arginine, asparagine, aspartic acid, glutamic acid, glutamine, lysine, serine, threonine, and tyrosine. Peptides are dipeptides, oligopeptides, and polypeptides, and the amino acids at both ends of the peptide are selected from the following: arginine, asparagine, aspartic acid, glutamic acid, glutamine, lysine, serine, threonine, tyrosine; the intermediate amino acid is selected from 20 arbitrary amino acids (for example, RGD).

### Embodiment 8: Preparation of targeting vector

Nano drug-delivery vector was prepared by using the basic nano-preparation material described in Embodiment 7, and then the targeting material in Embodiment 7 was attached to the surface of the prepared nano drug-delivery vector to obtain the targeting vector.

### Embodiment 9: Preparation of targeting element

The targeting element was synthesized by using the targeting material and spacer material described in Embodiment 7, and the synthesis of mannose and PEGn was taken as an example.

Diethylene glycol, p-toluenesulfonyl chloride and triethylamine were dissolved in DCM and reacted for 24 h at room temperature. ToSOC₂H₄OC₂H₄OH (Compound 1) was obtained by column chromatography. Compound 1 was dissolved in DCM with pentaacetylated mannose and its derivatives and boron trifluoride diethyl etherate (BF₃·Et₂O). Aco-M-OC₂H₄OC₂H₄OTos (Compound 2) was obtained by column chromatography, where M was mannose and its derivatives.

### Embodiment 10: Preparation of targeting vector

The synthesis was further carried out by using the target element in Embodiment 9 and the basic nano-preparation material. Here, the synthesis of mannose, PEGn and cholesterol was taken as an example.

The compound prepared in Embodiment 2 and sodium azide were dissolved in DMF, reacted at 60 °C for 24 h, and the solid product Aco-M-OC₂H₄OC₂H₄N₃(Compound 3) was obtained by silica gel column chromatography. Compound 3 was dissolved in methanol solution of sodium methoxide, reacted for 3 h at room temperature, and the product M-OC₂H₄OC₂H₄N₃ (Compound 4) was obtained by concentration. Cholesterol, bromopropyne and sodium-hydrogen were dissolved in the mixture of ether and DMF, reacted for 24 h at room temperature, and the solid product Chol-CH₂CCH (Compound 5) was obtained by silica gel column chromatography. Compound 4, Compound 5 and cuprous iodide were dissolved in DMF, reacted for 24 h at room temperature, and the solid product M-PEGₙ-Chol (Compound 6) was obtained by silica gel column chromatography.

### Embodiment 11: Improve the targetability of the targeting vector

The method of improving the targeting vector in Embodiment 8: A. synthesize the targeting element by using the targeting material and the spacer material in Embodiment 7; B. nano-preparations was prepared by using the basic nano-preparation material in Embodiment 7, and then link the targeting element obtained in step A with the nano drug-delivery vector in Embodiment 8.

### Embodiment 12: Synthesis of targeting vector

Mannose-PEGn-Chol: take the synthesis of mannose-PEG100-Chol as an example, and the sysnthesis was also conducted according to this synthetic route in case of other PEGs with different lengths. Diethylene glycol, p-toluenesulfonyl chloride and triethylamine were dissolved in DCM, reacted for 24 h at room temperature, and ToSOC₂H₄OC₂H₄OH (Formula I) was obtained by silica gel column chromatography. The compound shown in Formula I was dissolved in DCM with pentaacetylated mannose and boron trifluoride diethyl etherate (BF₃·Et₂O), reacted at room temperature for 24 h, and the product Aco-Mannose-OC2H4OC2H4OTos (Formula II) was obtained by silica gel column chromatography. The compound shown in Formula II and sodium azide were dissolved in DMF, reacted at 60°C for 24 h, and the solid product Aco-Mannose-OC2H4OC2H4N3 was obtained by silica gel column chromatography (Formula III). The compound shown in Formula III was dissolved in methanol solution of sodium methoxide, reacted at room temperature for 3 h, and the product Mannose-OC2H4OC2H4N3 (Formula IV) was obtained by concentration. Cholesterol, bromopropyne and sodium-hydrogen were dissolved in a mixed solution of ether and DMF, reacted at room temperature for 24 hours, and the solid product Chol-CH2CCH (Formula V) was obtained by silica gel column chromatography. The compounds shown in Formula IV, V and cuprous iodide were dissolved in DMF, reacted at room temperature for 24 h. The solid product Mannose-Chol (Formula VI) was obtained by silica gel column chromatography. The chemical shift δ (ppm) of each characteristic hydrogen proton in Figure 1 was analyzed: 5.30-5.42 ppm (1) is the hydrogen shift of =CH- in cholesterol; 7.92ppm (1) is hydrogen shift in N-CH=C. The presence of these characteristic peaks indicated that the compound shown in Formula IV was linked to the fragment of the compound in Formula V. The 1H-NMR results showed that the mannose-PEG100-Chol has been successfully coupled, please see Figure 1 for the detailed. The synthetic route is as follows:

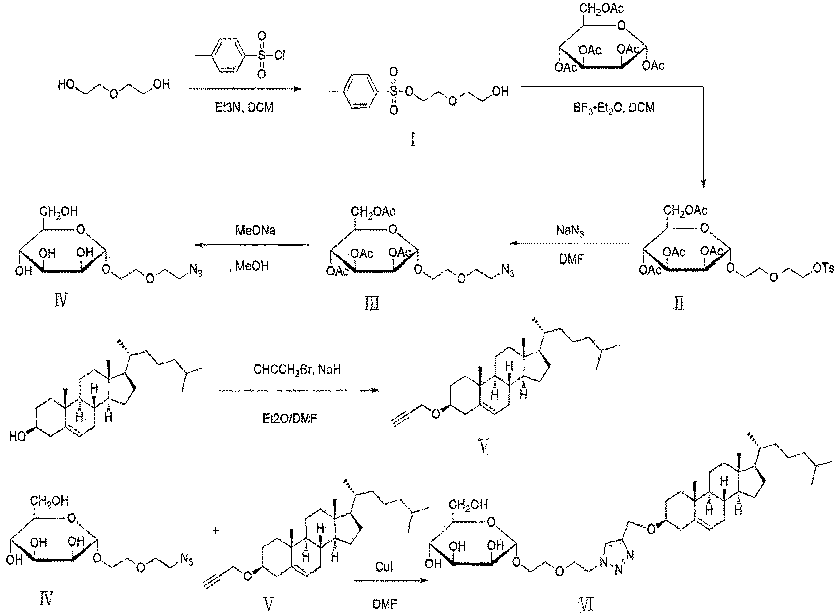

**Mannose-PEG2000-DSPE:** DSPE-PEG200-NH2 can be purchased directly. 5mg mannose was dissolved in 5ml ethanol. 5mg DSPE-PEG-NH2 was dissolved in 5ml chloroform-ethanol mixed solvent (9:1, V/V), and 0.5ml mannose ethanol solution was added dropwise under nitrogen protection; after mixed homogenously, 100µl triethylamine was added, with stirring at 40°C for 12h. After the reaction, the organic solvent was removed by rotary evaporation under reduced pressure, deionized water was added to redissolve. The unreacted mannose was removed by dialyzing, and the product mannose-PEG2000-DSPE was obtained by cryodesiccation.

**Synthesis of mannose-PEG2000-palmitic acid:** FmocNH-PEG-COOH was dissolved in water, with addition of EDC•HCl and sulfo-NHS and stirring at room temperature for 10 minutes. 2-aminoethyl-α-D-mannopyranoside was dissolved in water, and added to the above reaction system. The reaction was carried out at room temperature for 48 hours, and the product was obtained by silica gel column chromatography. Compound 1 was dissolved in DCM, followed by addition of 1-octanediol. The reaction lasted for several hours, and the product was obtained by silica gel column chromatography. Compound 2 and palmitic acid were added to a mixed solvent of ether and DMF, reacted at room temperature for several hours. The solid product mannose-PEG2000-palmitic acid was obtained by silica gel column chromatography, and the structural formula is as follows:

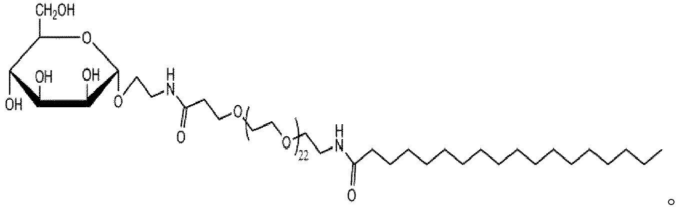

### Embodiment 13: Preparation of targeted drugs

The drug was encapsulated in the targeting vector prepared in Embodiment 8.

In the present embodiment, the drugs are small molecule drugs and/or protein polypeptide drugs and/or gene drug.

The small molecule drugs are anti-tumor drugs taxanes (Taxol, docetaxel, harringtonine, 7-epitaxol), camptothecins (camptothecin, SN38, irinotecan, 9-aminocamptothecin, 9-nitrocamptothecin, etc.), vinblastines (vinblastine, vincristine, vindesine, vinorelbine, vinflunine, etc.), doxorubicin, epirubicin, daunorubicin, epirubicin, amphotericin, methotrexate, cytarabine, 5-fluorouracil, mitoxantrone or its derivatives, gefitinib, noscapine, cisplatin, carboplatin, oxaliplatin, carmustine, quercetin, etc.

The main drug component protein polypeptide drugs are interleukins (for example, IL-1, IL-la, IL-2, IL-3, IL-4, IL-5, IL-6, etc.), various growth factors (for example, fibroblast growth factor, liver growth factor, vascular endothelial growth factor, hematopoietic growth factor, etc.), interferons (for example, IFNα, IFNβ, IFNγ), tumor necrosis factors (for example, TNFα, TNFβ), integrins, monoclonal antibodies, enzymes, insulin, etc.

The main drug component gene drug is DNA, plasmid, mRNA, siRNA, shRNA, microRNA, etc. When the targeting nano-preparations is used for immunotherapy, the main drug component can also encapsulate the immune adjuvant in addition to the drug used for immunotherapy to enhance the immune effect.

### Embodiment 14: Preparation of targeted drugs

| Formul ation | DOTA P (mg) | DOPC (mg) | Chol (mg) | Mannose -PEG100-Ch ol (mg) | Mannose -PEG 1000-Ch ol (mg) | Mannose -PEG2000-Ch ol (mg) | Mannose -PEG5000-Ch ol (mg) |
|---|---|---|---|---|---|---|---|
| Formulation 1 | 50 | 10 | 40 | 0 | 0 | 0 | 0 |
| Formulation 2 | 50 | 10 | 35 | 5 | 0 | 0 | 0 |
| Formulation 3 | 50 | 10 | 35 | 0 | 5 | 0 | 0 |
| Formulation 4 | 50 | 10 | 35 | 0 | 0 | 5 | 0 |
| Formulation 5 | 50 | 10 | 35 | 0 | 0 | 0 | 5 |

| Formulation | DOTA P (mg) | DSPE-PEG20 00(mg) | Chol (mg) | Mannose-PEG2000-DSPE (mg) | | Main drug | |
|---|---|---|---|---|---|---|---|
| Formulation 6 | 50 | 10 | 35 | 5 | | pGFP | 2mg |
| Formulation 7 | 50 | 10 | 35 | 2 | | PTX | 5mg |
| Formulation 8 | 50 | 10 | 35 | 1 | | Albumin | 4mg |
| Formulation 9 | 50 | 10 | 35 | 0 | | PTX | 2mg |
| Formulation 10 | 50 | 10 | 0 | 0 | | pGFP | 1mg |

| | | | | | | | |
|---|---|---|---|---|---|---|---|
| Note: the main drug of Formulation 1∼5 is GFP-mRNA (purchased from trilink company, batch number: WOTL6647). | | | | | | | |

**Preparation method 1:** according to the above formulation, the phospholipids with different weight proportion (when the main drug is PTX, it is added together with the phospholipids) were added into the round-bottom flask, dissolved in chloroform / ethanol (1:1 V / V). Then, the organic solvent was removed by rotary vaporation under reduced pressure, and a film was formed on the inner wall of the bottle. Subsequently, PBS 7.4 buffer solution was added for hydration, followed by 100 W ultrasonic treatment for 3 min to obtain liposomes. The blank liposome was incubated with pGFP at room temperature to encapsulate pGFP. The nanoparticles derived from mannose-PEG1000-Chol can be labeled as MP1000-LPX. The obtained liposomes were characterized, and the results showed that the particle size of the liposomes was 132.93±4.93 nm, and the zeta potential was 37.93±2.95 mV. It was also observed that the liposomes had obvious lipid membrane structure and the shape was similar to spherical (Figure 2-4).

**Preparation method 2 (Formulation 8):** the different proportions of phospholipids were dissolved with chloroform/ether according to the above formulation, and then the aqueous solution of albumin was added to obtain the emulsion by ultrasonic treatment. The organic solvent was removed by rotary evaporation. Subsequently, PBS 7.4 buffer solution was added to rehydrate the liposome.

### Embodiment 15: Preparation of targeted drug

Solid lipid nanoparticles are nano-structured vectors based on lipid as the framework material, which have the characteristics such as physiological compatibility, cell affinity and targeting, etc. In the present embodiment, addition of mannose modified lipid improved the targetability of solid lipid nanoparticles.

| | Oil phase (10ml) | | | | Aqueous phase (35ml) | | Main drug (DOX) (mg) |
|---|---|---|---|---|---|---|---|
| Formulation | Mannose-PE G2000-palmi tic acid (mg) | Glyceryl tristearate (mg) | Glyceryl monostear ate (mg) | Soybean lecithin (mg) | Poloxamer 188 (%) | Tween 80 (%) | |
| Formulation 11 | 0 | 0 | 40 | 60 | 0 | 2 | 10 |
| Formulation 12 | 0 | 0 | 40 | 60 | 3 | 0 | 10 |
| Formulation 13 | 20 | 0 | 80 | 0 | 3 | 0 | 10 |
| Formulation 14 | 20 | 80 | 0 | 0 | 2 | 0 | 10 |
| Formulation 15 | 50 | 50 | 0 | 0 | 2 | 0 | 10 |
| Formulation 16 | 50 | 50 | 0 | 0 | 4 | 0 | 10 |

**Preparation method:** the accurately weighed drugs and lipid materials according to the formulation were dissolved in ethanol to form the oil phase by stirring at a constant temperature in water bath; the formulated amount of surfactant was dissolved in purified water to form the aqueous phase by heating at a constant temperature in water bath; under stirring conditions, the oil phase was injected into the aqueous phase to emulsify and concentrate at a constant temperature. After the emulsion was concentrated to a certain volume, pour it into cold water at a constant temperature of 4°C for solidifying with stirring, followed by filtering with 0.45 µm membrane.

### Embodiment 16: Preparation of targeted drug

HDL is a kind of lipoprotein with heterogeneous composition, density and particle size. ApoA-I is the most abundant apolipoprotein in HDL, and its hydrophobic core transports cholesterol in the body. Recombinant high density lipoprotein is formed by the recombination of ApoA-I endogenously isolated or synthesized in vitro, phospholipid and cholesterol, and it is similar to endogenous HDL in biochemical characteristics and functions. High density lipoprotein has many excellent characteristics such as high safety, high efficiency of delivery (delivery drug in three ways: core-embedding, lipophilic drug embedding and recombinant high density lipoprotein core; surface insertion, and lipophilic drug insertion into the phospholipid monolayer; protein bonding), targetability. In the present embodiment, mannose ligand was added to the prepared HDL nanoparticles, and the targetability of the nanoparticles was further improved.

| Formulation | DOTAP (mg) | DOPC (mg) | DSPE-PEG2000 (mg) | Mannose-PEG2000-DSPE (mg) | Chol (mg) | Mannose-PEG 100 0-Chol (mg) | ApoA-I (mg) | Main drug (DOX) |
|---|---|---|---|---|---|---|---|---|
| Formulation 17 | 60 | 15 | 45 | 0 | 0 | 0 | 1 | 10mg |
| Formulation 18 | 0 | 120 | 0 | 0 | 0 | 0 | 1 | 10mg |
| Formulation 19 | 0 | 100 | 0 | 20 | 0 | 0 | 1 | 10mg |
| Formulation 20 | 0 | 100 | 0 | 20 | 0 | 0 | 1.5 | 10mg |
| Formulation 21 | 50 | 10 | 0 | 0 | 20 | 20 | 1 | 10mg |

| Formulation | DOTAP (mg) | DSPE-PEG20 00 (mg) | PLGA (mg) | Chol (mg) | Mannose-PEG1000-Chol (mg) | | ApoA-I (mg) | Main drug (siRNA/P TX) |
|---|---|---|---|---|---|---|---|---|
| Formulation 22 | 50 | 10 | 10 | 30 | 10 | | 0.5 | 2mg |
| Formulation 23 | 50 | 10 | 10 | 30 | 10 | | 1 | 2mg |
| Formulation 24 | 50 | 10 | 10 | 30 | 10 | | 1.5 | 2mg |
| Formulation 25 | 50 | 10 | 10 | 30 | 10 | | 2 | 2mg |

**Preparation method:** it is similar to the preparation of liposomes. The method suitable for preparing liposomes can be used for the preparation of HDL nanoparticles.

**Preparation method 1:** different proportions of phospholipid (main drug DOX liposoluble is added simultaneously) were added into a round-bottom flask according to the above formulation, and dissolved by chloroform. After the organic solvent was removed by rotary vaporation under reduced pressure, a layer of film was formed on the inner wall of the bottle. PBS7.4 buffer solution was added for hydration, followed by 100W ultrasonic treatment for 3min to obtain liposome. The formulated amount of ApoA-I was added into liposome injectable suspension slowly for stationary incubation at 4 °C for 24h, followed by dialyzing.

**Preparation method 2:** the different proportions of phospholipid were added into a round-bottom flask according to the above formulation, and dissolved by chloroform. After the organic solvent was removed by rotary vaporation under reduced pressure, a layer of film was formed on the inner wall of the bottle. Subsequently, PLGA/PTX nanoparticle injectable suspension was added, followed by 100W ultrasonic treatment for 3min to obtain liposome. The formulated amount of ApoA-I was added into liposome injectable suspension slowly for stationary incubation at 4 °C for 24h, followed by dialyzing.

### Embodiment 17: Preparation of targeted drug

| Formulation | Mannose-PEG-DSPE (mg) | DSPE (mg) | DOPC (mg) | DSPC (mg) | DMPC (mg) | Main drug |
|---|---|---|---|---|---|---|
| Formulation 26 | 20 | 60 | 20 | 0 | 0 | Quercet in (2mg) |
| Formulation 27 | 20 | 60 | 0 | 20 | 0 | DOX (5mg) |
| Formulation 28 | 20 | 60 | 35 | 0 | 20 | PTX (2mg) |
| Formulation 29 | 20 | 60 | 10 | 35 | 0 | DOX (5mg) |
| Formulation 30 | 50 | 0 | 50 | 0 | 0 | DOX (5mg) |

**Preparation method:** the vector material and main drug were dissolved in chloroform (other solvents such as ethanol or the mixed solvent of ethanol and chloroform can be selected according to the dissolution), and the organic solvent was removed by roatry vaporation under the reduced pressure. A thin film was formed on the inner wall of the bottle, and PBS 7.4 buffer solution was added for hydration.

### Embodiment 18: Uptake of targeting nanoparticles

12 hours before transfection, Raw264.7 cells in the plate were blown away, and the medium was re-suspended and counted. 1ml cell suspension with a density of 10 × 10⁴ / ml was placed into each well of 24-well plate, and incubated in the incubator for about 12 hours to be standby. After discarding the medium, lipopolysaccharide (LPS) with final concentration of 100 ng / mL was added into each well, and incubated at 37 °C for 12 hours to stimulate macrophages. After discarding the medium, different preparations with final concentration of 25 ng / mL (The experiments were conducted for all the formulations, Formulation 1 and Formulation 3were taken as examples) were separately added into each well, and incubated in dark at 37 °C for 2 hours to investigate the uptake of different preparations by macrophages. After reaching incubation time, discard the medium and wash twice with cold PBS; the cells in 24-well plate were collected by PBS, and then washed with PBS once (1200 rpm, 5 min), subsequently analyzed by flow cytometry.The results were shown in Figure 5 (from left to right: control, Formulation 3 and Formulation 1). It was found that the addition of targeting ligand mannose-PEG1000-Chol can significantly increase the transfection efficiency of the cells.

### Embodiment 19: Transfection efficiency of targeting nano-preparations

The DC2.4 was co-incubated with the prepared targeting nano-preparations in a 24-well plate, and the N/P ratio of the targeting nano-preparations was 5. The transfection efficiency and mean fluorescence intensity (MFI) of GFP-positive DC2.4 were detected by the flow cytometry. In short, DC2.4 was captured by forward scatter (FSC) and side scatter (SSC). The surviving DC2.4 was shown in Region 1 (R1), and the GFP-positive cells were shown in Region 2 (R2). The transfection efficiency was automatically displayed in R2. The MFI expressing GFP in GFP-positive cells was obtained by FlowJo software. It is necessary to subtract the background value of untreated DC2 for the calculation of MFI. Thereby, the in vitro transfection kinetics of the main drug mRNA was further understood, and the transfection efficiency of the mannose-PEG1000-Chol on DC2.4 (expressed at 12-72 h) was also confirmed.

As shown in Figure 6-7, the mannose-PEG1000-Chol group induced the most GFP-positive cells, and the proportion was up to 52%, which was significantly higher than any other group. The transfection efficiency of mannose-PEG1000-Chol was calculated as 52.09±4.85%, much exceeding the commercial transfection reagent Lipo 3K (11.47±2.31%).

### Embodiment 20: Stability test

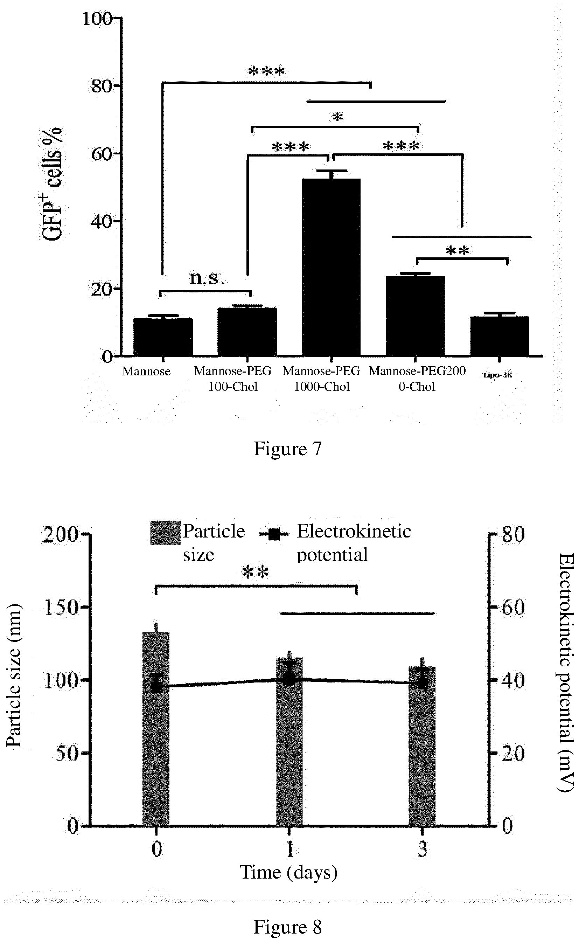

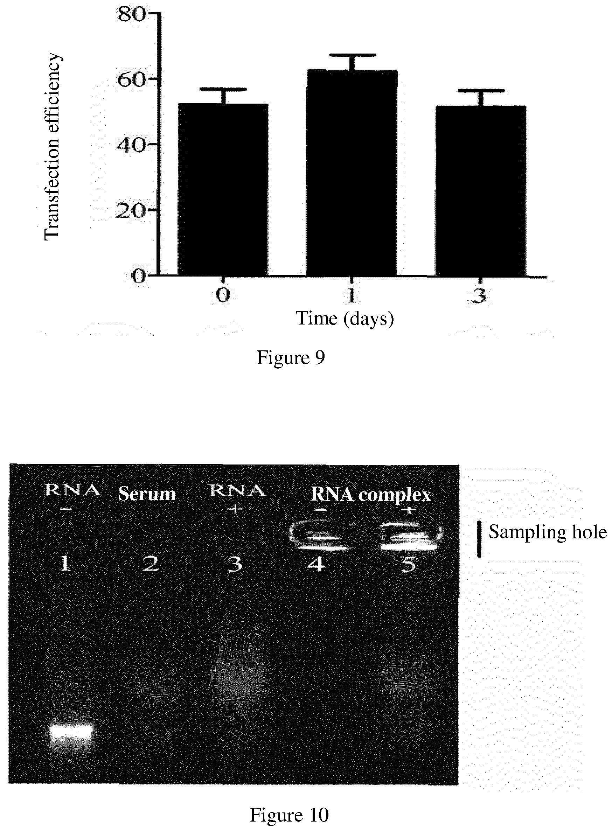

The stability of the mannose-PEG1000-Chol modified targeting nano-preparations was evaluated, and its stability was determined by the particle size, zeta potential and transfection efficiency. The results showed that the particle size of mannose-PEG1000-Chol modified targeting nano-preparations was slightly reduced, and the zeta potential did not decrease (Figure 8) when the mannose-PEG1000-Chol modified targeting nano-preparations was stored at 4 °C; if stored at 4 °C for 3 days, the transfection efficiency was about 50% (Figure 9). Please see the table below for details. Moreover, the mRNA incubated with mannose-PEG1000-Chol in the serum was not dissociated (Figure 10). Similarly, mannose-PEG100-Chol and mannose-PEG2000-Chol modified targeting nano-preparations also has good stability.

Transfection stability test results of mannose-PEG1000-Chol modified targeting nano-preparations:

| Time | Transfection efficiency |
|---|---|
| DAY 0 | 52.4±3.6 |
| DAY 1 | 61.7±3.8 |
| DAY 3 | 53.5±3.2 |

### Embodiment 21: Cytotoxicity test

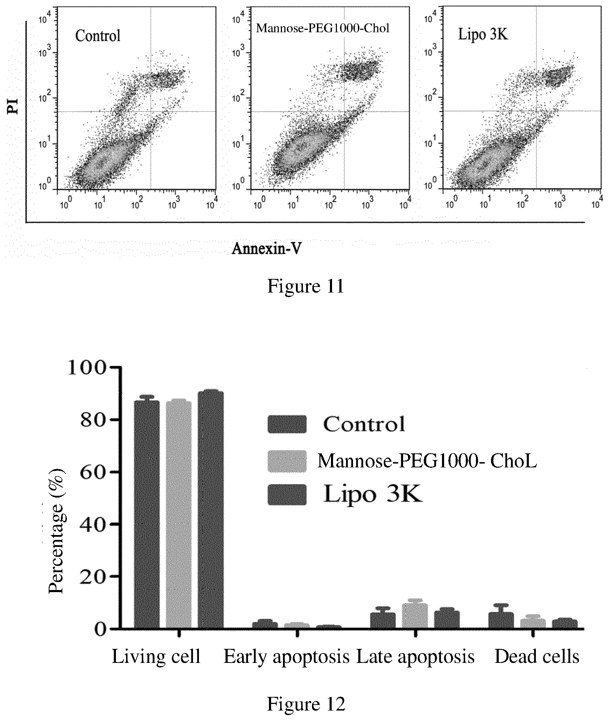

After 24-hour incubation with the specified formula, the cytotoxicity analysis was conducted by flow cytometry. The results were shown in Figure 11-12. The living cell rates of the control group, mannose-PEG1000-Chol group and Lipo 3K group were 86.7±3.6%, 86.7±1.7% and 90.1±1.2%, respectively, and no obvious difference was observed in early and late apoptosis, behaving well overall, without obvious cytotoxicity. Please see the table below for details:

| | Living cells | Early apoptosis | Late apoptosis | Necrosis |
|---|---|---|---|---|
| Control | 86.7±3.6 | 2.5±1.3 | 6.2±2.2 | 6.7±3.1 |
| Mannose-PEG 1000-Chol | 86.7±1.7 | 2.3±0.2 | 9.9±1.5 | 4.0±1.4 |
| Lipo 3K | 90.1±1.2 | 1.3±0.1 | 7.0±0.9 | 3.7±0.7 |

### Embodiment 22: DC2.4 uptake of targeting nanoparticles

12 hours before transfection, DC2.4 in the plate was blown away, and the medium was re-suspended and counted. 1ml cell suspension with a density of 10 × 10⁴ / ml was placed into each well of 24-well plate, and incubated in the incubator for about 12 hours to be standby. After discarding the medium, lipopolysaccharide (LPS) with final concentration of 100 ng / mL was added into each well, and incubated respectively at 37 °C and 4°C for 12 hours to stimulate DC2.4. After discarding the medium, different preparations with final concentration of 25 ng / mL (LPX and MP₁₀₀₀-LPX) were separately added into each well, and incubated respectively in dark at 37 °C and 4°C for 2 hours to investigate the DC2.4 uptake in case of different temperatures and preparations . After reaching incubation time, discard the medium and wash twice with cold PBS; the cells in 24-well plate were collected by PBS, and then washed with PBS once (1200 rpm, 5 min), subsequently analyzed by flow cytometry.

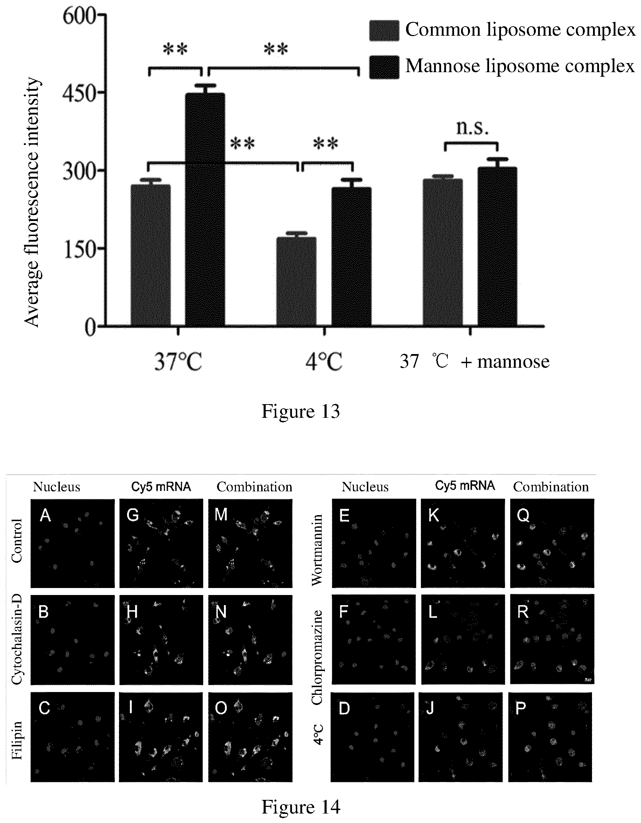

The results were shown in Figure 13. When the uptake temperature was reduced from 37 °C to 4 °C, the uptake of common mRNA liposome complex and mRNA targeted liposome complex decreased, indicating that the uptake of mRNA liposome complex by DC2.4 was energy-dependent, that is, the uptake reduced with the decrease of temperature. Compared with the data at 37 °C, it was found that DC2.4 could ingest more mRNA targeted liposome complexes. Compared with the uptake data with or without mannose, no significant change was shown in the uptake of common mRNA liposome complex by cells, but the uptake of mRNA targeted liposome complex by cells after addition of mannose was significantly reduced, indicating that the more uptake of mRNA targeted liposome complexes by DC2.4 is mainly mediated by mannose receptor. The detailed results were shown in the table below:

| | 37° | 4° | 37°+mannose |
|---|---|---|---|
| LPX | 274.2±8.7 | 172.1±7.9 | 284.2±6.3 |
| MP1000-L PX | 448.4±16.6 | 268.4±17.4 | 307.9±15.8 |

### Embodiment 22: BMDCs uptake of targeting nanoparticles

12 hours before transfection, BMDCs in the plate were blown away, and the medium was re-suspended and counted. 1ml cell suspension with a density of 10 × 10⁴ / ml was placed into each well of 24-well plate, and incubated in the incubator for about 12 hours to be standby. After discarding the medium, lipopolysaccharide (LPS) with final concentration of 100 ng / mL was added into each well, and separately incubated at 37 °C and 4 °C for 12 hours to stimulate BMDCs. After discarding the medium, the cells cultured at 37°C were divided into control group, cytochalasin-D group, filipin group, wortmannin group and chlorpromazine group, and MP₁₀₀₀-LPX with final concentration of 25 ng / mL were separately added to each group; MP₁₀₀₀-LPX was added to the cells cultured at 4°C. The above groups were separately incubated in dark at 37 °C and 4°C for 2 hours to investigate the BMDCs uptake in case of different temperatures and preparations . After reaching incubation time, discard the medium and wash twice with cold PBS; the cells in 24-well plate were collected by PBS, and then washed with PBS once (1200 rpm, 5 min), subsequently analyzed by flow cytometry.

As shown in Figures 14-15, the uptake decreased when the uptake temperature was lowered from 37°C to 4°C, indicating that the uptake of mRNA targeted liposome complexes by BMDCs was energy-dependent, that is, the uptake reduces as the temperature decreases, which was consistent with the DC2.4 result. Comparing the uptake data with or without endocytosis inhibitors, it was found that there was no obvious change in the uptake of mRNA targeted liposome complexes by cells after addition of cytochalasin-D and filipin, but the uptake of mRNA targeted liposome complexes by cells was significantly reduced after addition of wortmannin and chlorpromazine, indicating that the uptake of mRNA targeted liposome complexes by BMDCs is not only energy-dependent, but also affected by pinocytosis (wortmannin) and clathrin (chlorpromazine) mediated endocytosis. The relevant results are shown in the table below:

| Test conditions | Uptake result (×104) |
|---|---|
| Control | 2.52±0.16 |
| Cytochalasin-D | 2.73±0.15 |
| Filipin | 2.61±0.15 |
| 4° | 1.29±0.25 |
| Wortmannin | 1.47±0.17 |
| Chloropromazine | 0.42±0.10 |

### Embodiment 23: In vivo anti-tumor results of MP400-LPX

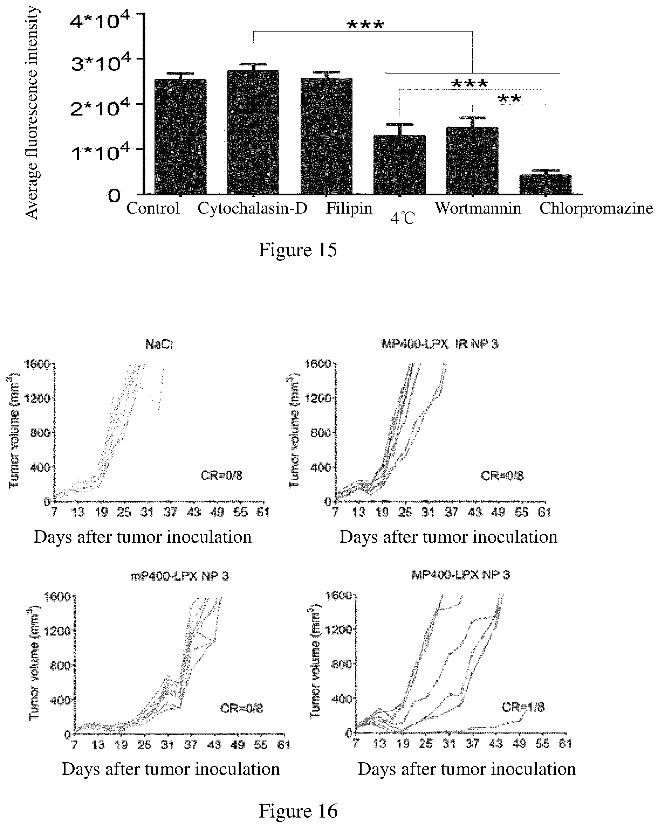

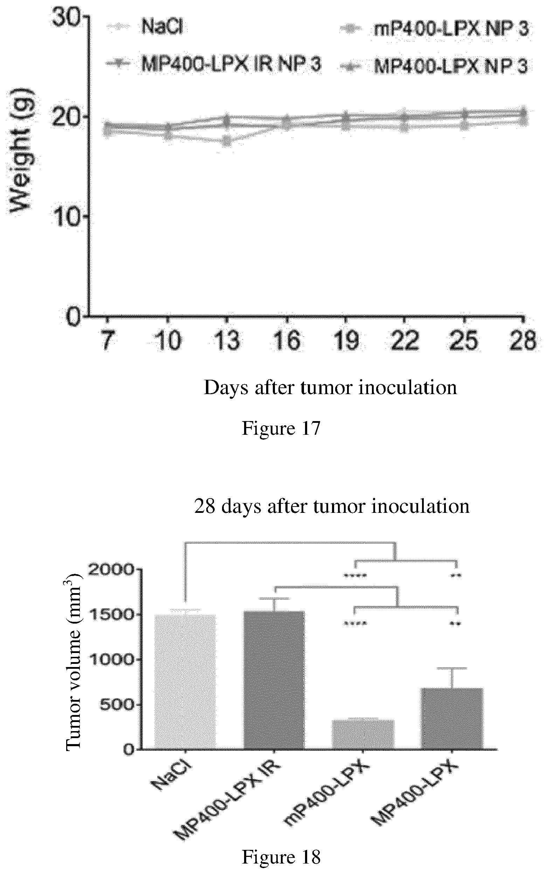

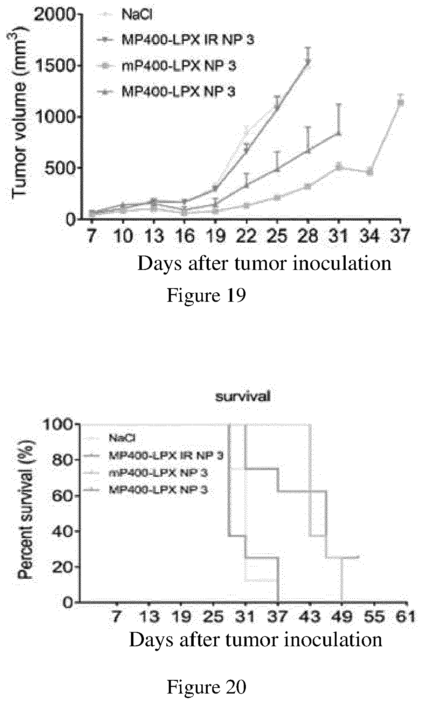

MP400-LPX and methoxypolyethylene glycol 400 cholesterol (mP400-LPX) was synthesized according to the methods in Embodiment 7 and Embodiment 9, containing the main component E6/E7-mRNA and forming the mannose targeted E6/E7-mRNA liposome complex. On Day 0, 3, 10, and 17, mice were injected with 30ug of the complex, and the results were shown in Figures 16-20. Compared with the control group and the un-correlated mRNA group, the tumor growth data of mice in the mannose targeted E6/E7-mRNA liposome complex group was significantly reduced. It should be noted that the mice inoculated with vaccine derived from mRNA-loaded liposomes prepared by mannose polyethylene glycol 400 cholesterol have a longer survival time, compared with the control material of methoxypolyethylene glycol 400 cholesterol, and the tumors of one mouse completely disappeared, indicating that the vaccine prepared by the targeting preparations has better immunotherapy effect.

### Embodiment 24 Uptake of MP400-LPX by DC2.4 cells

12 hours before transfection, DC2.4 and BMDCs in the plate were blown away separately, and the medium was re-suspended and counted. 1ml cell suspension with a density of 10 × 10⁴ / ml was placed into each well of 24-well plate, and incubated in the incubator for about 12 hours to be standby. After discarding the medium, lipopolysaccharide (LPS) with final concentration of 100 ng / mL was added into each well, and incubated at 37 °C for 12 hours to stimulate DC 2.4 and BMDCs. After discarding the medium, divide each well into six groups, add different Cy5-H-mRNA-loaded preparations (control, LPX, MP₁₀₀-LPX, MP₄₀₀-LPX, MP₁₀₀₀-LPX and MP₂₀₀₀-LPX) with a final concentration of 25 ng/mL to the DC2.4, and incubate in dark at 37 °C for 2 hours to investigate the DC 2.4 uptake in case of different preparations . Meanwhile, add different GFP-mRNA-loaded preparations (LPX, MP₁₀₀-LPX, MP₄₀₀-LPX, MP₁₀₀₀-LPX, MP₂₀₀₀-LPX and Lipo 3K) with a final concentration of 25 ng/mL to the DC2.4 and BMDCs, and incubate in dark at 37 °C for 2 hours to investigate the transfection effect of the DC2.4 and BMDCs in case of different preparations . After reaching incubation time, discard the medium and wash twice with cold PBS; the cells in 24-well plate were collected by PBS, and then washed with PBS once (1200 rpm, 5 min), subsequently analyzed by flow cytometry.

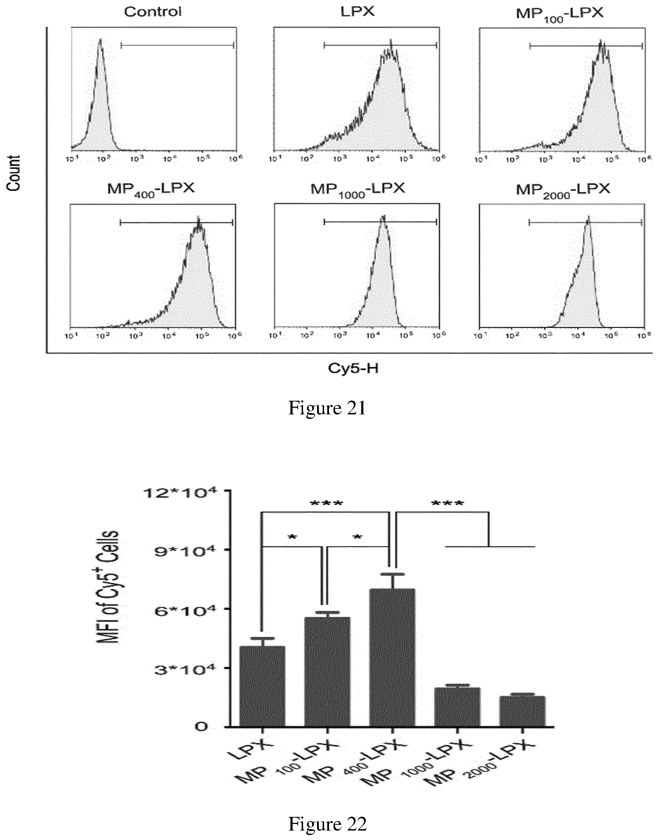

The results were shown in Figure 21-22. DC2.4 had the best uptake of MP400-LPX. The uptake efficiency of DC2.4 for mRNA-loaded targeting liposome complexes with different ligand chain lengths was about 100%. However, the uptake of each preparation by DC2.4 is different, which was specifically displayed in the intracellular fluorescence intensity. Compared with LPX, MP100-LPX intracellular accumulation was significantly increased; MP400-LPX intracellular accumulation was significantly increased compared with MP100-LPX; MP1000-LPX intracellular accumulation has significantly decreased compared with MP400-LPX. The intracellular accumulation degree of MP1000-LPX and MP2000-LPX was similar. Compared with other preparations such as LPX, MP100-LPX, MP1000-LPX and MP2000-LPX, MP400-LPX intracellular accumulation was significantly increased. The above results indicated that the uptake of mRNA-loaded targeting liposome complexes with different ligand chain lengths by DC2.4 was affected by the chain length of the ligand. The suitable chain length or chain length range allows DC2.4 to ingest more.

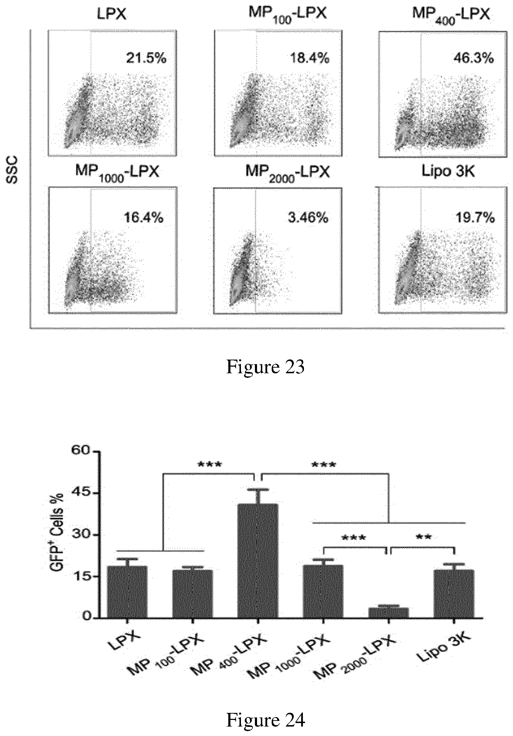

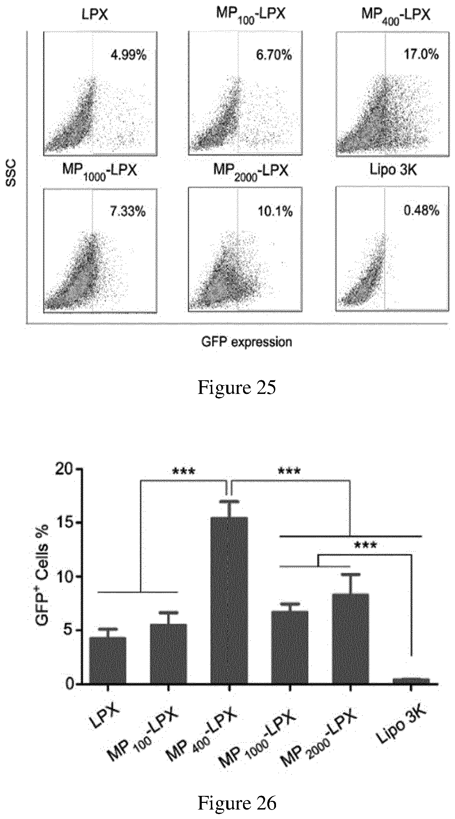

As shown in Figure 23-26, DC2.4 transfected MP400-LPX was best. The transfection efficiency of DC2.4 for mRNA-loaded targeting liposome complexes with different ligand chain lengths was different, which was specifically manifested in the different percentages of GFP-positive cells. Compared with LPX, MP100-LPX, MP1000-LPX MP2000-LPX, MP400-LPX showed a significant increase in GFP-positive cells, and MP2000-LPX had a significant decrease in GFP-positive cells compared with other preparations .The transfection results of BMDCs were basically similar to that of DC2.4, but unlike DC2.4, MP2000-LPX and other preparations has significantly higher transfection efficiency than than Lipo 3K. BMDCs are primary cells, and this transfection result indicated that targeting preparations are expected to have good application prospects.

### Embodiment 25: In vivo anti-tumor experiment of mRNA-loaded targeting liposome complexes with different ligand chain lengths

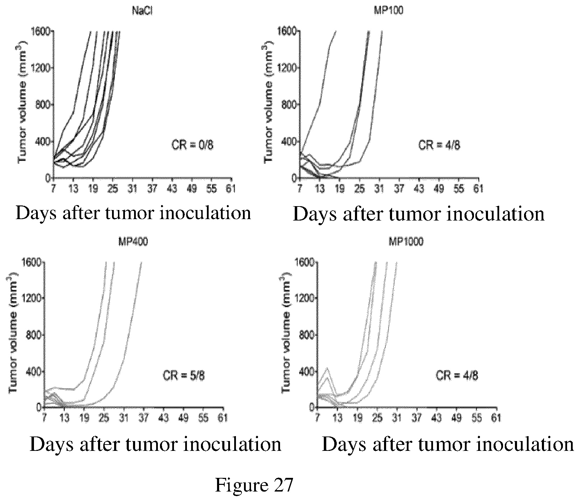

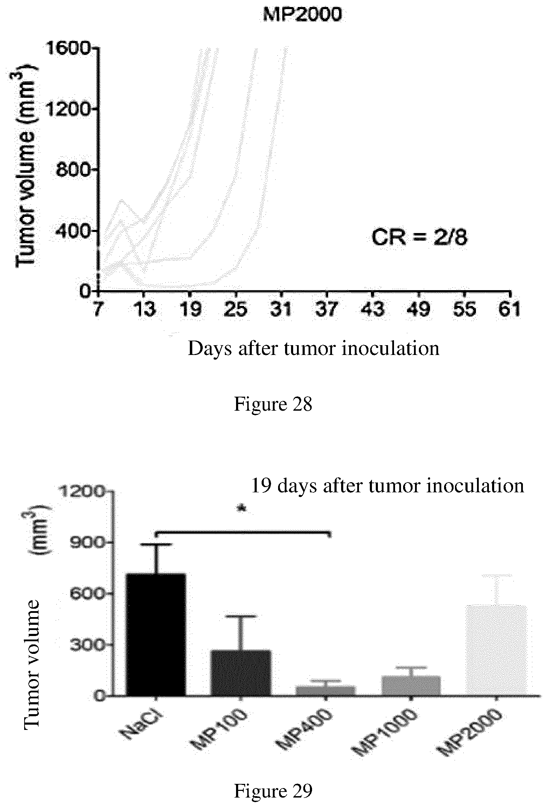

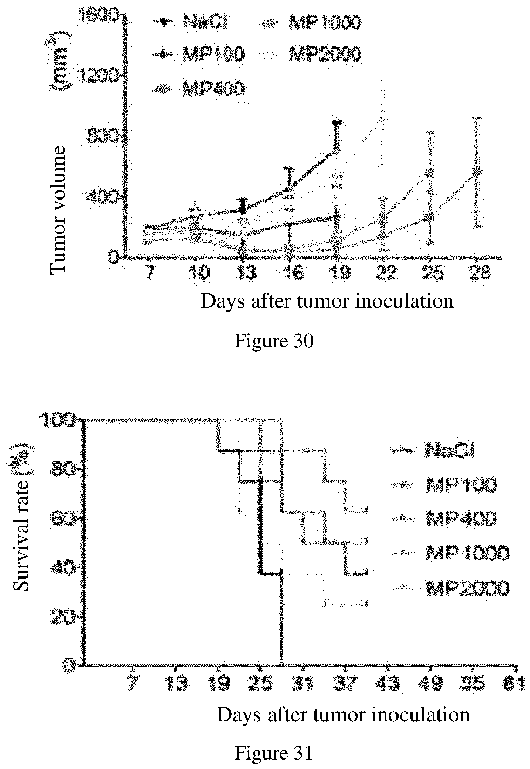

MP100-LPX, MP400-LPX, MP1000-LPX and MP2000-LPX were synthesized according to the methods in Embodiment 7 and Embodiment 9, containing the main component E6/E7-mRNA and forming the mannose targeted E6/E7-mRNA liposome complex. On Day 0, 3, 10, and 17, mice were injected with 30ug of the complex. The results are shown in Figure 27-31. MP400-LPX showed the best effect. Compared with other chain-length targeting ratios, the mannose cholesterol targeting ligand of polyethylene glycol 400 exhibited the best anti-tumor effect in vivo. Specifically, the mouse had the highest tumor cure rate, up to 60%. Moreover, the growth rate of tumors in uncured mice was lower, and the survival time of tumor-bearing mice can be extended.

Finally, it should be noted that the above embodiments are only used to illustrate the technical schemes of the present invention and not to limit them. Although the present invention has been described in detail with reference to the preferred embodiments, the ordinary technicians in the art should understand that the technical schemes of the present invention can be modified or equivalently replace without departing from the objective and scope of the technical scheme of the present invention, which should be covered by the scope of the claims of the present invention.

## Claims

1. A targeting nano-preparations modified by mannose and its derivatives, wherein composing of targeting ligand mannose and its derivatives, nano-preparations and main drug components, which can be used for vaccine delivery, targeted cancer therapy, and treatment of arteriosclerosis and various inflammatory diseases.

2. The targeting nano-preparations according to claim 1, wherein the mannose derivative is one or more of mannoside, mannosamine, mannan, etc.

3. The targeting nano-preparations according to claim 1, wherein the nano-preparations is one or more of liposomes, emulsions, nanogels, core-shell nanoparticles, HDL nanoparticles, solid lipid nanoparticles, polymeric micelles, etc.

4. The targeting nano-preparations according to claim 1, wherein the main drug component is one or more of small molecule drugs, protein polypeptide drugs or gene drug, etc.

5. The targeting nano-preparations according to claim 1, wherein the content of the targeting ligand mannose and its derivatives is 0.05% - 40%.

6. The targeting nano-preparations according to claim 1, wherein the content of the targeting ligand mannose and its derivatives is 1% - 10%.

7. The targeting nano-preparations according to claim 1, wherein the content of the targeting ligand mannose is 2% - 5%.

8. The targeting nano-preparations according to claim 1, wherein the modification method of the targeting ligand mannose and its derivatives is as follows: after preparing the nano-preparations, the mannose and its derivatives are adsorbed or coupled to the surface of the nano-preparations; alternatively, the mannose and its derivatives are coupled with the vector material for preparing the nano-preparations, and then the nano-preparations is prepared.

9. The targeting nano-preparations according to claim 8, wherein the mannose and its derivatives are adsorbed on the surface of the nano-preparations by solvent evaporation method, high pressure homogenization method or microemulsion method.

10. The targeting nano-preparations according to claim 1, wherein the mannose and its derivatives used for modifying the nano-preparations are single mannose and its derivatives or 2-10 mannose and its derivatives.

11. The targeting nano-preparations according to claim 8, wherein the coupling mode of the mannose and its derivatives with the nano-preparations or the vector material for preparing the nano-preparations is direct coupling or coupling through a spacer.

12. The targeting nano-preparations according to claim 11, wherein the spacer is one or more of glutaraldehyde, ethylenediamine, malonic acid, PEG, amino acid, dipeptide, oligopeptide, polypeptide, stearoyl, palmitoyl, etc.

13. The targeting nano-preparations according to claim 1-12, wherein the vector materials for preparing nano-preparations at least have one of the active amino or hydroxyl groups, which are pharmaceutically acceptable for preparing micelles, emulsions, nanogels, core-shell nanoparticles, HDL nanoparticles, solid lipid nanoparticles, liposomes, polymeric micelles, ect., and are prepared by the preparation method for pharmaceutically acceptable nano-preparations.

14. A composition for preparing nano-preparations with targeting function, wherein the composition comprises targeting material and basic nano-preparation material, and the targeting material is mannose and / or mannose derivative.

15. The composition according to claim 14, wherein the mannose derivative is mannoside and / or mannosamine and / or mannan.

16. The composition according to claim 15, wherein the mannose derivatives are methyl-D-mannoside, 1-α formylmethyl-mannopyranoside, 4-aminophenyl-α-D-mannopyranoside, 4-nitrophenyl-α-D-mannopyranoside, 4- methylumbelliferyl -α-D-mannopyranoside, mannose-6-phosphate and carbamoyl-D-mannose.

17. The composition according to claim 14 or 15 or 16, wherein the weight percentage of the targeting material in the composition is 0.05-40%.

18. The composition according to claim 14 or 15 or 16, wherein the weight percentage of the targeting material in the composition is 1-10%.

19. The composition according to claim 14, wherein the composition is composed of the targeting material, the basic nano-preparation material, and the spacer material which makes the targeting material and the basic nano-preparation material produce a distance, and the spacer material is one or more of glutaraldehyde, ethylenediamine, malonic acid, short alkyl chain, PEG, amino acid, dipeptide, oligopeptide, polypeptide, stearoyl and palmitoyl.

20. The composition according to claim 14, wherein the basic nano-preparation material is a composition for preparing liposomes, emulsions, nanogels, core-shell nanoparticles, HDL nanoparticles, solid lipid nanoparticles and polymeric micelles.

21. The composition according to claim 19, wherein the spacer material is PEGn, where n = 100-5000.

22. The composition according to claim 21, wherein n is 100, 200, 300, 400, 500, 600, 800, 900, 1000 or 2000.

23. The composition according to claim 19, wherein it is composed of 1 mannose, 0.5-56 PEG and 2-11 cholesterol based on weight.

24. The composition according to claim 19, wherein it is composed of 1 mannose, 10-25 PEG and 4-9 cholesterol based on weight.

25. The targeting vector for drug delivery is prepared by the composition according to claim 14.

26. The targeting vector according to claim 25, wherein the drug is a small molecule drug and / or a protein polypeptide drug and / or gene drug.

27. The targeting vector according to claim 26, wherein the small molecule drug is one ore more of taxane, camptothecin, vinblastine, adriamycin, epirubicin, daunorubicin, epirubicin, amphotericin, methotrexate, cytarabine, 5-fluorouracil, mitoxantrone or its derivatives, gefitinib, noscaline, cisplatin, carboplatin, oxaliplatin, carmustine and quercetin.

28. The targeting vector according to claim 26, wherein the protein polypeptide drug is interleukin, growth factor, interferon, integrin, monoclonal antibody, enzyme and insulin.

29. The targeting vector according to claim 28, wherein the interleukin includes but is not limited to IL-1, IL-la, IL-2, IL-3, IL-4, IL-5 and IL-6; the growth factors include but are not limited to fibroblast growth factor, liver growth factor, vascular endothelial growth factor and hematopoietic growth factor, and the interferon includes but is not limited to IFN α, IFN β and IFN γ, and the said interferon includes but is not limited to IFN α, IFN β and IFN γ; the tumor necrosis factors include but are not limited to TNF α and TNF β.

30. The targeting vector according to claim 26, wherein the gene drug is one or more of DNA, plasmid, mRNA, siRNA, shRNA and microRNA.

31. The drugs coated by the targeting vector described in claim 25.

32. The preparation method of the targeting vector according to claim 31, wherein the basic nano-preparation material is prepared into a nano drug-delivery vector, and the targeting material is attached to the surface of the nano drug-delivery vector.

33. The method for improving targetability of the targeting vector according to claim 25, wherein:
A Syntheze targeting element
Synthesizing the targeting material and the spacer material into a targeting element;
B Prepare targeting vector
The basic nano-preparation material is prepared into a nano drug-delivery vector, and then the targeting element obtained in Step A is linked with the nano drug-delivery vector to obtain the targeting vector; alternatively, the spacer material is first synthesized with the basic nano-preparation material, and then further linked with the targeting material to obtain the targeting vector.

34. The method according to claim 33, wherein the targeting material is mannose and its derivatives, and the spacer material is PEG.

35. The method according to claim 33, wherein the distance between the targeting element and the nano drug-delivery vector is 0.2-100nm.

36. The method according to claim 35, wherein the distance between the targeting element and the nano drug-delivery vector is 0.3-10nm.

37. The method for delivering the effective ingredient to the target cell, wherein the effective ingredient is placed in the targeting vector described in claim 25 and transported.

38. The method according to claim 37, wherein the target cell contains mannose receptor.

39. The method according to claim 37, wherein the target cells include but are not limited to macrophages, dendritic cells and tumor cells.

40. A composition for preparing a targeting element, wherein the composition is composed of targeting material and spacer material, and the targeting material is mannose and / or mannose derivative.

41. The composition according to claim 40, wherein the mannose derivative is mannoside and / or mannosamine and / or mannan.

42. The composition according to claim 40, wherein the mannose derivative is any one or more of methyl-D-mannoside, 1-α formylmethyl-mannopyranoside, 4-aminophenyl-α-D-mannopyranoside, 4-nitrophenyl-α-D-mannopyranoside, 4-methylumbelliferyl-α-D-mannopyranoside, mannose-6-phosphate and carbamoyl-D-mannose.

43. The composition according to claim 40, wherein the spacer material is one or more of glutaraldehyde, ethylenediamine, malonic acid, short alkylchain, PEG, amino acid, dipeptide, oligopeptide, polypeptide, stearyl and palmitoyl.

44. The composition according to claim 40, wherein the molar ratio of the spacer material to the targeting material is 1-10:1.

45. The composition according to claim 44, wherein the molar ratio of the spacer material to the targeting material is 5:1.

46. The composition according to claim 40, wherein the molar ratio of mannose to PEG is 1:5.

47. The composition according to claim 46, wherein the molar ratio of mannose to PEG is 1:1.

48. The targeting element is prepared by using the composition described in claim 40.

49. The targeting element according to claim 48, wherein the targeting element is shown in Formula I, wherein R1 is -C₅H₈O₂, -C₂H₇N₂, -C₃H₃O₄, -O(CH₂CH₂O)nH, -C₂H₃O₂NR' or -C₂H₃ONR', -C₄H₅O₃N₂R₁' R₂' or -C₄H₅O₂N₂R₁' R₂', -C₃n+1H₃n+2On+2Nn+1R₁' R₂' or -C₃n+1H₃n+2On+1Nn+1R₁' R₂', -C₁₈H₃₅O, C₁₆H₃₁O, -H , -CHO, -C₆H₄NH₂, - C₆H₄NO₂, -CONH₂, -CH₂C₆CCHCO₂CH₃, both R₁'and R₂' are R' groups of any natural amino acid side chain, R₂ is - CH₃ or - PO₃H₂, R₃ is - H, R₄ is - H, R₅ is - H or -NHCOCH₃,

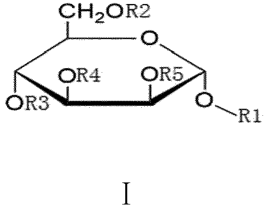

50. The targeting element according to claim 49, wherein R1 is polyethylene glycol and the structure is 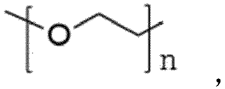 where n = 1-5000, the corresponding molecular weight is 40 * n.

51. The targeting vector contains the targeting element described in claim 48.

52. The targeting vector according to claim 51, wherein it is linked by a targeting element and nano-preparations. The nano-preparations are liposomes, emulsions, nanogels, core-shell nanoparticles, HDL nanoparticles, solid lipid nanoparticles and polymeric micelles.

53. The targeting vector according to claim 51, wherein 1-100000 targeting elements are linked to a single targeting vector.

54. The targeting vector according to claim 53, wherein 20-1000 targeting elements are linked to a single targeting vector.

55. The targeting vector according to claim 52, wherein the distance between the target head of the targeting element and the nano-preparations is 0.2-100nm.

56. The targeting vector according to claim 55, wherein the distance between the target head of the targeting element and the nano-preparations is 0.3-10nm.

57. The targeting vector according to claim 55 or 56, wherein the target head is mannoside and / or mannamine and / or mannan.

58. The method for linking the targeting element described in claim 48 to the nano-preparations, wherein the targeting material and the spacer material are linked and then linked with the nano-preparations material to prepare the nano-preparations.

59. The method for linking the targeting element described in claim 48 to the nano-preparations, wherein it specifically comprises the following steps:
1) Diethylene glycol, p-toluenesulfonyl chloride and triethylamine are dissolved in DCM and reacts at room temperature, and Compound 1 is obtained by column chromatography;
2) Compound 1 is dissolved in DCM with pentaacetylated mannose and its derivatives and boron trifluoride ether. The Compound 2 is obtained by column chromatography;
3) Compound 2 and sodium azide are dissolved in DMF and Solid compound 3 is obtained by silica gel column chromatography;
4) Compound 3 is dissolved in methanol solution of sodium methoxide, reacts at room temperature and Compound 4 is obtained by concentration;
5) Cholesterol, bromopropyne and sodium-hydrogen are dissolved in the mixed solution of ether and DMF, reacts at room temperature. Compound 5 is obtaimned by silica gel column chromatography;
6) Compound 4, Compound 5 and cuprous iodide are dissolved in DMF and reacts for 24 h at room temperature. The solid compound 6 of targeting vector is obtained by silica gel column chromatography.

60. The method for improving the drug delivery capacity of the targeting vector described in claim 51, wherein the drug is encapsulated in the targeting vector for delivery.

61. The method according to claim 60, wherein the drug is a small molecule drug and / or a protein polypeptide drug and / or a gene drug.

62. The method according to claim 60, wherein the targeting vector can deliver the drug to a target cell containing a mannose receptor.

63. A complex containing the targeting vector described in claim 51, wherein the complex is formed by the targeting vector and the receptor, the targeting vector delivers drugs, and the receptor is mannose.

64. The method for improving the efficiency of the transfection reagent, wherein the composition of the targeting element described in claim 40 is linked with the vector for preparing the transfection reagent to prepare the transfection reagent with high efficiency.

65. The targeting element described in claim 48 is used as a target oil phase.

66. The targeting element described in claim 48 is used as a target aqueous phase.
